# EUROPEAN PATENT APPLICATION

(11) **EP 4 219 650 A1**
(43) Date of publication of application: **02.08.2023**
(21) Application number: 21872358.3
(22) Date of filing: 17.09.2021
(51) Int. Cl.: C09K 9/02, C09D 201/00, C09D 11/037, C09D 11/17, C09D 11/326, B43K 5/00, B43K 7/00, B43K 8/02, B43K 29/02, C09D 7/41, C09B 11/28, C09B 67/08, C09B 67/20

(54) **REVERSIBLE THERMOCHROMIC COMPOSITION AND REVERSIBLE THERMOCHROMIC MICROCAPSULE PIGMENT ENCAPSULATING SAME**

(30) Priority: 28.09.2020 JP 2020162412
(71) Applicant: THE PILOT INK CO., LTD., Nagoya-shi, Aichi 466-8588 (JP); Kabushiki Kaisha Pilot Corporation (also trading as Pilot Corporation), Tokyo 104-8304 (JP)
(72) Inventor: UMEMOTO, Hiroshi, Nagoya-shi, Aichi 466-8588 (JP); MAEHASHI, Kaori, Nagoya-shi, Aichi 466-8588 (JP); MASUSHIGE, Naoto, Tokyo 104-8304 (JP); MORIGAKI, Yui, Tokyo 104-8304 (JP)
(74) Representative: Elkington and Fife LLP
(86) International application number: PCT/JP2021/034302
(87) International publication number: WO 2022/065229

(57) **Abstract**

[Problems] To provide a reversibly thermochromic composition having excellent contrast between a colored state and a decolored state, and excellent light resistance, and a reversibly thermochromic microcapsule pigment encapsulating the reversibly thermochromic composition.

[Solution] Disclosed is a reversibly thermochromic composition including: (a) an electron-donating color-developing organic compound; (b) a combination of a compound having a specific structure as an electron-accepting compound; and (c) a reaction medium which reversibly induces an electron transfer reaction between the component (a) and the component (b) in a specific temperature range, and a reversibly thermochromic microcapsule pigment encapsulating the reversibly thermochromic composition.

## Description

### TECHNICAL FIELD

The present invention relates to a reversibly thermochromic composition and a reversibly thermochromic microcapsule pigment encapsulating the same. More specifically, the present invention relates to a reversibly thermochromic composition that exhibits a decolored state in a temperature range not lower than an upper color changing point and exhibits a colored state in a temperature range not higher than a lower color changing point, and a reversibly thermochromic microcapsule pigment encapsulating the reversibly thermochromic composition.

### BACKGROUND ART

There has been disclosed a reversibly thermochromic composition containing, as essential components, an electron-donating color-developing organic compound, an electron-accepting compound, and a reaction medium which reversibly induces an electron transfer reaction between the electron-donating color-developing organic compound and the electron-accepting compound in a specific temperature range and showing a color change from a colored state to a decolored state. For example, Patent Literature 1 discloses the reversibly thermochromic composition described above, and particularly discloses that a compound having a specific structure is used alone or in combination of two or more kinds for a color developer which is an electron-accepting compound.

The reversibly thermochromic composition is required to have a high density in the colored state, a low density in the decolored state, and excellent contrast. The reversibly thermochromic composition is further required to have excellent light resistance during color development, that is, to suppress a decrease in color development density with time even when exposed to light in the colored state.

### CITATION LIST

### PATENT LITERATURE

Patent Literature 1: JP 2000-297277 A

### SUMMARY OF THE INVENTION

### OBJECT OF THE INVENTION

The present invention has been made based on the background art as described above, and its object is to provide a reversibly thermochromic composition which has a high density in a colored state, a low density in a decolored state, and excellent light resistance, and a reversibly thermochromic microcapsule pigment encapsulating the reversibly thermochromic composition.

### SOLUTION TO PROBLEM

A reversibly thermochromic composition according to the present invention includes
(a) an electron-donating color-developing organic compound,
(b) a combination of a compound represented by Formula (I) and a compound represented by Formula (IIa) or (IIb) as an electron-accepting compound, and
(c) a reaction medium which reversibly induces an electron transfer reaction between the component (a) and the component (b) in a specific temperature range: 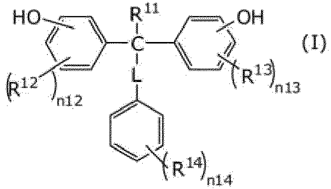
wherein
R¹¹ is a hydrogen atom or a linear or branched alkyl group having 1 to 6 carbon atoms,
L is a single bond, a linear or branched alkylene group having 1 to 3 carbon atoms, or an aryl-substituted alkylene group having 7 to 9 carbon atoms,
R¹² and R¹³ are each independently a linear or branched alkyl group having 1 to 4 carbon atoms, which may be substituted by a fluorine atom, a cyclic alkyl group having 3 to 7 carbon atoms, a linear or branched alkoxy group having 1 to 3 carbon atoms, an alkenyl group having 2 to 4 carbon atoms, an aryl group having 6 to 10 carbon atoms, or a halogen atom,
R¹⁴ is each independently a linear or branched alkyl group having 1 to 4 carbon atoms, which may be substituted by a fluorine atom, a cyclic alkyl group having 3 to 7 carbon atoms, a linear or branched alkoxy group having 1 to 3 carbon atoms, an alkenyl group having 2 to 4 carbon atoms, an aryl group having 6 to 10 carbon atoms, which may be substituted by a halogen atom or a hydroxy group, an aryl-substituted alkyl group having 7 to 11 carbon atoms, which may be substituted by a halogen atom or a hydroxy group, a hydroxy group, or a halogen atom,
n12 and n13 are each independently 0 to 3, and
n14 is 0 to 3, 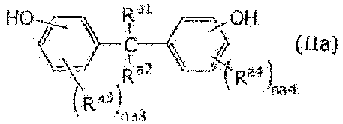 wherein
R^{a1} and R^{a2} are each independently a hydrogen atom or a linear or branched alkyl group having 1 to 17 carbon atoms (where a methylene (-CH₂-) group in the alkyl group may be replaced with an oxy (-O-) group or a carbonyl (-CO-) group), which may be substituted by a fluorine atom,
R^{a3} and R^{a4} are each independently a linear or branched alkyl group having 1 to 4 carbon atoms, which may be substituted by a fluorine atom or a hydroxy group, an alkenyl group having 2 to 4 carbon atoms, or a halogen atom, and
na3 and na4 are each independently 0 to 2, 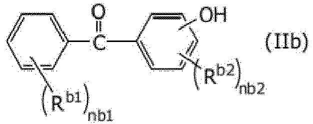 wherein
R^{b1} and R^{b2} are each independently a hydroxy group, a linear or branched alkoxy group having 1 to 9 carbon atoms, a linear or branched alkyl group having 1 to 10 carbon atoms, which may be substituted by a fluorine atom, an alkenyl group having 2 to 10 carbon atoms, or a halogen atom,
nb1 is 0 to 3, and
nb2 is 0 to 2.

A reversibly thermochromic microcapsule pigment according to the present invention encapsulates the reversibly thermochromic composition described above.

A reversibly thermochromic liquid composition according to the present invention includes the reversibly thermochromic microcapsule pigment described above and a vehicle.

A reversibly thermochromic solid writing material or solid cosmetic material according to the present invention includes the reversibly thermochromic microcapsule pigment described above and an excipient.

A resin composition for forming a reversibly thermochromic molded article according to the present invention includes the reversibly thermochromic microcapsule pigment described above and a molding resin.

A reversibly thermochromic laminate according to the present invention includes a support and a reversibly thermochromic layer containing the reversibly thermochromic microcapsule pigment.

A writing instrument according to the present invention stores ink for writing instrument including a reversibly thermochromic microcapsule pigment and a vehicle.

### ADVANTAGEOUS EFFECTS OF THE INVENTION

According to the present invention, an object is to provide a reversibly thermochromic composition which has a high density in a colored state, a low density in a decolored state, excellent contrast between the colored state and the decolored state, and excellent light resistance, and a reversibly thermochromic microcapsule pigment encapsulating the reversibly thermochromic composition.

### BRIEF DESCRIPTION OF DRAWINGS

FIG. 1 is a graph for explaining a hysteresis characteristic of a reversibly thermochromic composition of heat-decoloring type in a color density-temperature curve.
FIG. 2 is a graph for explaining the hysteresis characteristic of the reversibly thermochromic composition of heat-decoloring type having a color-memory property in the color density-temperature curve.
FIG. 3 is a graph for explaining the hysteresis characteristic of a reversibly thermochromic composition of heat color-developing type in the color density-temperature curve.

### BEST MODE FOR CARRYING OUT THE INVENTION

Examples of a reversibly thermochromic composition of the present invention include a reversibly thermochromic composition of heat-decoloring type (decolored when heated, and colored when cooled) including at least three essential components: (a) an electron-donating color-developing organic compound; (b) an electron-accepting compound; and (c) a reaction medium to determine an occurrence temperature of a coloring reaction of the component (a) and the component (b).

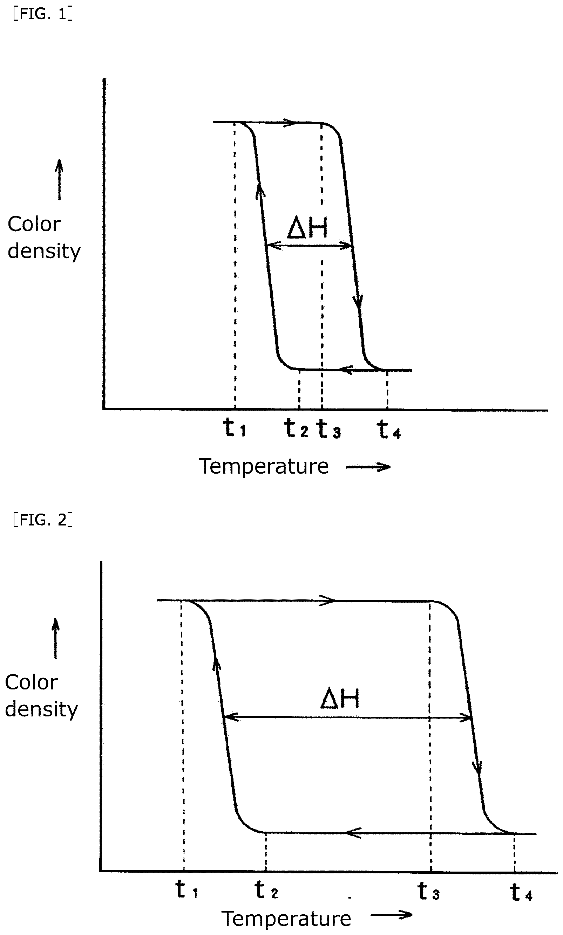

As the reversibly thermochromic composition described above, it is possible to use a reversibly thermochromic composition described in JP S51-44706 B, JP S51-44707 B, JP H1-29398 B, etc. and being of heat-decoloring type (decolored when heated, and colored when cooled) which changes color above and below a predetermined temperature (color changing point), exhibits a decolored state in a temperature range not lower than an upper color changing point, exhibits a colored state in a temperature range not higher than a lower color changing point, and has characteristics in which only one specific state, of the both states, exists in a normal temperature range, and the other state is maintained only while heat or cold required for the other state to be expressed is being applied, but the state in the normal temperature range is restored once the application of heat or cold is terminated and in which a hysteresis width (ΔH) is relatively small (ΔH = 1 to 7°C) (see FIG. 1).

Also, it is possible to use a reversibly thermochromic composition of heat-decoloring type (decolored when heated, and colored when cooled) which is described in JP H4-17154 B, JP H7-179777 A, JP H7-33997 A, JP H8-39936A, JP 2005-1369 A, etc., exhibits a characteristic of large hysteresis width (ΔH = 8 to 70°C), changes the color along very different paths in the curve of plots showing color development density change with temperature change between when the temperature increases from a region lower than the discoloration range and when the temperature decreases from a region higher than the discoloration range, and has color-memory property when the colored state in a temperature range not higher than a complete coloring temperature t₁ or the decolored state in a high-temperature range not lower than a complete decoloring temperature t₄ is in the specific temperature range [temperature range between a coloring starting temperature t₂ and a decoloring starting temperature t₃ (essentially two-phase retaining temperature range)] (see FIG. 2).

The respective components (a), (b), and (c) will be specifically explained below.

The component (a), namely, an electron-donating color-developing organic compound, is a color-determining component and is a compound which develops a color by donating an electron(s) to the component (b), which is a color developer.

Examples of the electron-donating color-developing organic compound include phthalide compounds, fluoran compounds, styrynoquinoline compounds, diazarhodamine lactone compounds, pyridine compounds, quinazoline compounds, and bisquinazoline compounds.

Examples of the phthalide compounds include diphenylmethane phthalide compounds, phenylindolyl phthalide compounds, indolyl phthalide compounds, diphenylmethane azaphthalide compounds, phenylindolyl azaphthalide compounds, and derivatives of these compounds, among which phenylindolyl azaphthalide compounds and their derivatives are preferred.

Examples of the fluoran compounds include aminofluoran compounds, alkoxyfluoran compounds, and derivatives of these compounds.

Hereinafter, compounds that can be used for the component (a) are exemplified.
3,3-bis(4-dimethylaminophenyl)-6-dimethylaminophthalide,
3-(4-diethylaminophenyl)-3-(1-ethyl-2-methylindol-3-yl)phthalide,
3,3-bis(1-n-butyl-2-methylindol-3-yl)phthalide,
3,3-bis(2-ethoxy-4-diethylaminophenyl)-4-azaphthalide,
3-(2-ethoxy-4-diethylaminophenyl)-3-(1-ethyl-2-methylindol-3-yl)-4-azaphthalide,
3-(2-n-hexyloxy-4-diethylaminophenyl)-3-(1-ethyl-2-methylindol-3-yl)-4-azaphthalide,
3-[2-ethoxy-4-(N-ethylanilino)phenyl]-3-(1-ethyl-2-methylindol-3-yl)-4-azaphthalide,
3-(2-acetamide-4-diethylaminophenyl)-3-(1-propyl-2-methylindole-3-yl)-4-azaphthalide,
3,6-bis(diphenylamino)fluoran,
3,6-bis(N-phenyl-N-p-tolylamino)fluoran,
3,6-dimethoxyfluoran,
3,6-di-n-butoxyfluoran,
2-methyl-6-(N-ethyl-N-p-tolylamino)fluoran,
3-chloro-6-cyclohexylaminofluoran,
2-methyl-6-cyclohexylaminofluoran,
2-chloroamino-6-di-n-butylaminofluorane,
2-(2-chloroanilino)-6-di-n-butylaminofluoran,
2-(3-trifluoromethylanilino)-6-diethylaminofluoran,
2-(3-trifluoromethylanilino)-6-di-n-pentylaminofluorane,
2-dibenzylamino-6-diethylaminofluoran,
2-(N-methylanilino)-6-(N-ethyl-N-p-tolylamino)fluoran,
1,3-dimethyl-6-diethylaminofluora n,
2-chloro-3-methyl-6-diethylaminofluoran,
2-anilino-3-methyl-6-diethylaminofluoran,
2-anilino-3-methoxy-6-diethylaminofluoran,
2-anilino-3-methyl-6-di-n-butylaminofluoran,
2-anilino-3-methoxy-6-di-n-butylaminofluoran,
2-xyridino-3-methyl-6-diethylaminofluoran,
2-anilino-3-methyl-6-(N-ethyl-N-p-tolylamino)fluoran,
6-diethylamino-1,2-benzofluorane,
6-(N-ethyl-N-isobutylamino)-1,2-benzofluorane,
6-(N-ethyl-N-isopentylamino)-1,2-benzofluorane,
2-(3-methoxy-4-dodecoxystyryl)quinoline,
2-diethylamino-8-diethylamino-4-methylspiro[5H-[1]benzopyrano[2,3-d]pyrimidine-5,1'(3'H)-isobenzofuran]-3'-one,
2-di-n-butylamino-8-di-n-butylamino-4-methylspiro[5H-[1]benzopyrano[2,3-d]pyrimidine-5,1'(3'H)-isobenzofuran]-3'-one,
2-di-n-butylamino-8-diethylamino-4-methylspiro[5H-[1]benzopyrano[2,3-d]pyrimidine-5,1'(3'H)-isobenzofuran]-3'-one,
2-di-n-butylamino-8-(N-ethyl-N-isoamylamino)-4-methylspiro[5H-[1]benzopyrano[2,3-d]pyrimidine-5,1'(3'H)-isobenzofuran]-3'-one,
2-di-n-butylamino-8-di-n-pentylamino-4-methylspiro[5H-[1]benzopyrano[2,3-d]pyrimidine-5,1'(3'H)-isobenzofuran]-3'-one,
4,5,6,7-tetrachloro-3-(4-dimethylamino-2-methoxyphenyl)-3-(1-n-butyl-2-methyl-1H-indol-3-yl)-1(3H)-isobenzofuranone,
4,5,6,7-tetrachloro-3-(4-diethylamino-2-ethoxyphenyl)-3-(1-ethyl-2-methyl-1H-indol-3-yl)-1(3H)-isobenzofuranone,
4,5,6,7-tetrachloro-3-(4-diethylamino-2-ethoxyphenyl)-3-(1-n-pentyl-2-methyl-1H-indol-3-yl)-1(3H)-isobenzofuranone,
4,5,6,7-tetrachloro-3-(4-diethylamino-2-methylphenyl)-3-(1-ethyl-2-methyl-1H-indol-3-yl)-1(3H)-isobenzofuranone,
3',6'-bis[phenyl(2-methylphenyl)amino]spiro[isobenzofuran-1(3H),9'-[9H]xanthen]-3-one,
3',6'-bis[phenyl(3-methylphenyl)amino]spiro[isobenzofuran-1(3H),9'-[9H]xanthen]-3-one,
3',6'-bis[phenyl(3-ethylphenyl)amino]spiro[isobenzofuran-1(3H),9'-[9H]xanthene]-3-one,
2,6-bis(2'-ethyloxyphenyl)-4-(4'-dimethylaminophenyl)pyridine,
2,6-bis(2',4'-diethyloxyphenyl)-4-(4'-dimethylaminophenyl)pyridine,
2,6-bis(2,4-diethyloxyphenyl)-4-[4-bis(4-methyloxyphenyl)a minophenyl] pyridi ne,
2-(4'-dimethylaminophenyl)-4-methoxyquinazoline, and
4,4'-ethylenedioxy-bis[2-(4-diethylaminophenyl)quinazoline].

The fluorans may be compounds which contain a substituent in a xanthene ring-forming phenyl group, and in addition, may also be compounds which have a blue or black color and which contain a substituent in a xanthene ring-forming phenyl group as well as in a lactone ring-forming phenyl group (these substituents may be, for example, an alkyl group such as a methyl group or a halogen atom such as a chlorine atom).

The component (b), namely an electron-accepting compound, is a compound which receives an electron(s) from the component (a) and functions as a color developer of the component (a).

The component (b) is a combination of a compound represented by Formula (I) and a compound represented by Formula (IIa) or (IIb).

The compound represented by Formula (I) is as follows. 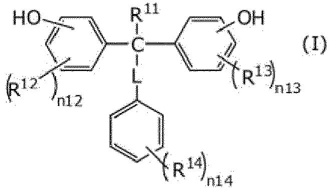 Wherein
R¹¹ is a hydrogen atom or a linear or branched alkyl group having 1 to 6 carbon atoms, preferably a hydrogen atom or a methyl group.

L is a single bond, a linear or branched alkylene group having 1 to 3 carbon atoms, or an aryl-substituted alkylene group having 7 to 9 carbon atoms, preferably a single bond or an ethylene group, and more preferably a single bond.

In the present invention, the aryl group also includes an alkylsubstituted aryl group (for example, a tolyl group). The same applies to the following aryl groups.

R¹² and R¹³ are each independently a linear or branched alkyl group having 1 to 4 carbon atoms, which may be substituted by a fluorine atom, a cyclic alkyl group having 3 to 7 carbon atoms, a linear or branched alkoxy group having 1 to 3 carbon atoms, an alkenyl group having 2 to 4 carbon atoms, an aryl group having 6 to 10 carbon atoms, or a halogen atom, and are preferably a linear or branched alkyl group having 1 to 4 carbon atoms (more preferably a methyl group), a linear or branched alkoxy group having 1 to 3 carbon atoms (more preferably a methoxy group or an ethoxy group), a cyclohexyl group, or a halogen atom (more preferably a fluorine atom).

R¹⁴ is each independently a linear or branched alkyl group having 1 to 4 carbon atoms, which may be substituted by a fluorine atom, a cyclic alkyl group having 3 to 7 carbon atoms, a linear or branched alkoxy group having 1 to 3 carbon atoms, an alkenyl group having 2 to 4 carbon atoms, an aryl group having 6 to 10 carbon atoms, which may be substituted by a halogen atom or a hydroxy group, an aryl-substituted alkyl group having 7 to 11 carbon atoms, which may be substituted by a halogen atom or a hydroxy group, a hydroxy group, or a halogen atom,
preferably a linear or branched alkyl group having 1 to 4 carbon atoms, which may be substituted by a fluorine atom, a cyclic alkyl group having 3 to 7 carbon atoms, a linear or branched alkoxy group having 1 to 3 carbon atoms, an aryl-substituted alkyl group having 7 to 11 carbon atoms, which is substituted by a hydroxy group, or a hydroxy group,
more preferably a methyl group, an ethyl group, a cyclohexyl group, a methoxy group, an ethoxy group, a hydroxyphenyl-substituted propyl group, or a hydroxy group, and
still more preferably hydroxyphenyl-substituted propyl group or a hydroxy group.
n12 and n13 are each independently 0 to 3, preferably each 0 or 1, and more preferably each 0. n14 is 0 to 3, preferably 0 or 1. In a more preferred embodiment, each hydroxy group is present at the 4-position (para position) of a benzene ring.

Specific examples of (I) include the following:
1-phenyl-1,1-bis(4-hydroxyphenyl)methane,
1-phenyl-1,1-bis(4-hydroxyphenyl)ethane,
4,4',4"-methylidinetrisphenol,
4,4'-[(4-hydroxyphenyl)methylene]bis(2-methylphenol),
4,4'-[(4-hydroxyphenyl)methylene]bis(2-cyclohexyl-5-methylphenol),
4,4',4"-ethylidinetrisphenol,
4,4',4"-ethylidinetris(2-methylphenol),
4,4'-[1-{4-[1-(4-hydroxyphenyl)-1-methylethyl]phenyl}ethylidene]bisphenol,
4,4'-[1-{4-[1-(4-hydroxy-3-methylphenyl)-1-methylethyl]phenyl}ethylidene]bis(2-methylphenol),
4,4'-[(3-ethoxy-4-hydroxyphenyl)methylene]bisphenol,
4,4'-[3-(5-cyclohexyl-4-hydroxy-2-methylphenyl)-3-phenyl)propylidene]bis(2-cyclohexyl-5-methylphenol),
4,4'-[(4-hydroxyphenyl)methylene]bis(2-isopropylphenol),
4,4'-[1-{4-[1-(3-fluoro-4-hydroxyphenyl)-1-methylethyl]phenyl}ethylidene] bisphenol,
4,4'-[3-(2,5-dimethyl-4-hydroxyphenyl)butylene]bis(2,5-dimethylphenol),
1,1,3-tris(4-hydroxyphenyl)propane,
1,2,2-tris(4-hydroxyphenyl)propane,
1,3,3-tris(4-hydroxyphenyl)butane,
1,4,4-tris(4-hydroxyphenyl)penta ne,
2,2-bis(3-methyl-4-hydroxyphenyl)-1-(4-hydroxyphenyl)propane,
1-(3-methyl-4-hydroxyphenyl)-2,2-bis(4-hydroxyphenyl)propane,
1-(3-methyl-4-hydroxyphenyl)-3,3-bis(4-hydroxyphenyl)butane,
3,3-bis(3-methyl-4-hydroxyphenyl)-1-(4-hydroxyphenyl)butane,
1,1,3-tris(3-methyl-4-hydroxyphenyl)propane,
1,3,3-tris(3-methyl-4-hydroxyphenyl)butane, and
4,4'-[4-(4-hydroxyphenyl)-sec-butylidene]bis(2-methylphenol).

The compound represented by Formula (IIa) is as follows. 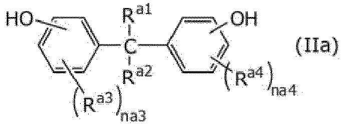 Wherein
R^{a1} and R^{a2} are each independently a hydrogen atom or a linear or branched alkyl group having 1 to 17 carbon atoms (where a methylene (-CH₂-) group in the alkyl group may be replaced with an oxy (-O-) group or a carbonyl (-CO-) group), which may be substituted by a fluorine atom,
preferably, R^{a1} and R^{a2} are each independently a hydrogen atom or a linear or branched alkyl group having 1 to 11 carbon atoms, which may be substituted by a fluorine atom,
more preferably, at least one of R^{a1} and R^{a2} is a linear or branched alkyl group having 5 to 9 carbon atoms, and
still more preferably, each independently, one of R^{a1} and R^{a2} is a branched alkyl group having 5 to 9 carbon atoms, and the other is a hydrogen atom or a methyl group.

R^{a3} and R^{a4} are each independently a linear or branched alkyl group having 1 to 4 carbon atoms, which may be substituted by a fluorine atom or a hydroxy group, an alkenyl group having 2 to 4 carbon atoms, or a halogen atom, and preferably a linear or branched alkyl group having 1 to 4 carbon atoms (more preferably a methyl group) or a halogen atom (more preferably a fluorine atom).

na3 and na4 are each independently 0 to 2, preferably each 0 or 1, and more preferably each 0. In a more preferred embodiment, each hydroxy group is present at the 4-position (para position) of a benzene ring.

Specific examples of (IIa) include the following:
1,1-bis(4-hydroxyphenyl)n-hexane,
1,1-bis(4-hydroxyphenyl)n-octane,
1,1-bis(4-hydroxyphenyl)n-decane,
1,1-bis(4-hydroxyphenyl)-2-methylpropane,
1,1-bis(4-hydroxyphenyl)-2-ethylbutane,
1,1-bis(4-hydroxyphenyl)-2-ethylhexane,
2,2-bis(4-hydroxyphenyl)propane,
2,2-bis(4-hydroxyphenyl)n-heptane,
2,2-bis(4-hydroxyphenyl)n-dodecane,
2,2-bis(4-hydroxyphenyl)-4-methylhexane,
2,2-bis(4-hydroxyphenyl)hexafluoropropane,
2,2-bis(4-hydroxy-3-methylphenyl)butane,
2,2-bis(4-hydroxy-3-isopropylphenyl)propane,
2,2-bis(3-fluoro-4-hydroxyphenyl)propane.

The compound represented by Formula (IIb) is as follows. 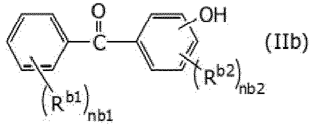 Wherein
R^{b1} and R^{b2} are each independently a hydroxy group, a linear or branched alkoxy group having 1 to 9 carbon atoms, a linear or branched alkyl group having 1 to 10 carbon atoms, which may be substituted by a fluorine atom, an alkenyl group having 2 to 10 carbon atoms, or a halogen atom,
preferably a hydroxy group or a linear or branched alkyl group having 1 to 8 carbon atoms,
more preferably a hydroxy group or a linear or branched alkyl group having 3 to 6 carbon atoms, and
still more preferably a hydroxy group, an isobutyl group, a sec-butyl group, or a tert-butyl group.
nb1 is 0 to 3, preferably 1. nb2 is 0 to 2, preferably 1. In a more preferred embodiment, the hydroxy group is present at the 2-position (ortho position) and the 4-position (para position) of one benzene ring.

Specific examples of (IIb) include the following:
2,4-dihydroxybenzophenone,
4,4'-dihydroxybenzophenone,
2,4-dihydroxy-4'-n-propylbenzophenone,
2,4-dihydroxy-4'-n-butylbenzophenone,
2,4-dihydroxy-4'-isobutylbenzophenone,
2,4-dihydroxy-4'-sec-butylbenzophenone,
2,4-dihydroxy-4'-tert-butylbenzophenone,
2,4-dihydroxy-4'-n-hexylbenzophenone,
2,4-dihydroxy-2',4'-dimethylbenzophenone,
2,4-dihydroxy-2',4',6'-trimethylbenzophenone,
2,4-dihydroxy-2'-methoxybenzophenone,
2,4-dihydroxy-4'-ethoxybenzophenone,
2,4-dihydroxy-2',4'-dimethoxybenzophenone, and
2,4-dihydroxy-3',4'-diethoxybenzophenone.

By combining the compound represented by Formula (I) and the compound represented by Formula (IIa) or (IIb) as the component (b), the properties of each compound can be utilized, and a reversibly thermochromic composition having a high color development density and excellent light resistance in the colored state can be provided.

Among the compounds represented by Formulas (IIa) and (IIb), the compound represented by Formula (IIa) is preferable from the viewpoint of easy availability and excellent productivity, that is, as the component (b), a combination of the compound represented by Formula (I) and the compound represented by Formula (IIa) is preferable from the viewpoint of color development density, light resistance, and productivity.

Among the compounds represented by Formula (I), preferred is a compound in which when R¹¹ is a hydrogen atom or a methyl group, L is a single bond or an ethylene group, n12 and n13 are each 0, n14 is 0 or 1, and n14 is 1, R¹⁴ is a methyl group, an ethyl group, a methoxy group, an ethoxy group, a hydroxyphenyl-substituted propyl group, or a hydroxy group, and each hydroxy group is present at the 4-position (para position) of the benzene ring, from the viewpoint that it is easy to obtain a reversibly thermochromic composition having an excellent color development density. More preferred is a compound in which when R¹¹ is a hydrogen atom or a methyl group, L is a single bond, n12 and n13 are each 0, n14 is 0 or 1, and n14 is 1, R¹⁴ is a hydroxyphenyl-substituted propyl group or a hydroxy group, and each hydroxy group is present at the 4-position (para position) of the benzene ring.

As compared with a case where the compound represented by Formula (I) is used alone as the component (b), the decolor density of the reversibly thermochromic composition can be maintained at the same level, or the decolor density can be lowered, so that the compound represented by Formula (IIa) to be combined with the compound represented by Formula (I) is preferably a compound in which at least one of R^{a1} and R^{a2} is a linear or branched alkyl group having 5 to 9 carbon atoms, na3 and na4 are each 0, and each hydroxy group is present at the 4-position (para position) of the benzene ring. The compound represented by Formula (IIa) is more preferably a compound in which each independently, one of R^{a1} and R^{a2} is a branched alkyl group having 5 to 9 carbon atoms, the other is hydrogen atom or a methyl group, na3 and na4 are each 0, and each hydroxy group is present at the 4-position (para position) of the benzene ring.

That is, most preferred is a combination of a compound, as the compound represented by Formula (I), in which when R¹¹ is a hydrogen atom or a methyl group, L is a single bond, n12 and n13 are each 0, n14 is 0 or 1, and n14 is 1, R¹⁴ is a hydroxyphenyl-substituted propyl group or a hydroxy group, and each hydroxy group is present at the 4-position (para position) of the benzene ring, and a compound, as the compound represented by Formula (IIa), in which each independently, one of R^{a1} and R^{a2} is a branched alkyl group having 5 to 9 carbon atoms, the other is a hydrogen atom or a methyl group, n3 and n4 are each 0, and each hydroxy group is present at the 4-position (para position) of the benzene ring.

A mass ratio of the compound represented by Formula (I) : the compound represented by Formula (IIa) is in a range of preferably 1 : 0.5 to 1 : 5, and more preferably 1 : 1 to 1 : 3.

When the mass ratio of the compound represented by Formula (I) and the compound represented by Formula (IIa) is in the above range, it is easy to obtain a reversibly thermochromic composition having a large difference between the color density and the decolor density, that is, being excellent in contrast between the colored state and the decolored state. When the mass ratio of the compound represented by Formula (I) : the compound represented by Formula (IIa) is in the range of 1 : 1 to 1 : 3, the compound represented by Formula (IIa) can easily dissolve the compound represented by Formula (I), can further improve the color development density of the reversibly thermochromic composition, and can provide a reversibly thermochromic composition in which the contrast between the colored state and the decolored state is more excellent.

The component (c) of the reaction medium which reversibly induces an electron transfer reaction between the component (a) and the component (b) in a specific temperature range will be described.

Examples of the component (c) include alcohols, esters, ketones, ethers, and acid amides.

When the reversibly thermochromic composition of the present invention is applied to microencapsulation and secondary processing, since low molecular weight ones evaporate out of a capsule when subjected to high heat treatment, in order to stably hold the composition inside the capsule, a compound having 10 or more carbon atoms is preferably used.

As the alcohols, aliphatic monovalent saturated alcohols having 10 or more carbon atoms are effective.

As the esters, esters having 10 or more carbon atoms are effective, and examples include esters obtained from optional combinations of aliphatic and aliphatic ring- or aromatic ring-containing monovalent carboxylic acids and aliphatic and aliphatic ring- or aromatic ring-containing monohydric alcohols, esters obtained from optional combinations of aliphatic and aliphatic ring- or aromatic ring-containing polyvalent carboxylic acids and aliphatic and aliphatic ring- or aromatic ring-containing monohydric alcohols and esters obtained from optional combinations of aliphatic and aliphatic ring- or aromatic ring-containing monovalent carboxylic acids and aliphatic and aliphatic ring- or aromatic ring-containing polyhydric alcohols.

In addition, an ester compound selected out of an ester between a saturated fatty acid and a branched aliphatic alcohol, an ester between an unsaturated fatty acid, or a saturated fatty acid having a branch or a substituent, and an aliphatic alcohol that is branched or has 16 or more carbon atoms; and cetyl butyrate, stearyl butyrate, and behenyl butyrate, is also preferable.

In addition, in order to exhibit a large hysteresis characteristic with respect to a color density-temperature curve to cause discoloration and to impart a color-memory property depending on a temperature change, a carboxylic acid ester compound having a ΔT value (melting point - cloud point) of 5°C or higher and less than 50°C described in JP 4-17154 B can be exemplified.

A fatty acid ester compound obtained from an aliphatic monohydric alcohol having an odd number not less than 9 of carbon atoms, and an aliphatic carboxylic acid having an even number of carbon atoms, and a fatty acid ester compound with a total carbon number of 17 to 23 to be obtained from n-pentyl alcohol or n-heptyl alcohol and an aliphatic carboxylic acid having an even number from 10 to 16 of carbon atoms, are also effective.

As the ketones, aliphatic ketones having a total carbon number of 10 or more are effective, and aryl alkyl ketones having a total carbon number of 12 to 24 can be mentioned.

As the ethers, aliphatic ethers having a total carbon number of 10 or more are effective.

Examples of the alcohols, esters, ketones, ethers, and acid amides include compounds described in JP 2020-100710 A.

As the component (c), a compound expressed by the following Formula (1) may be used. 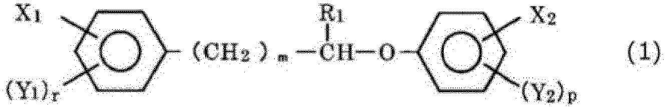 [wherein R₁ represents a hydrogen atom, or a methyl group, m represents an integer of 0 to 2, one of X₁ and X₂ represents -(CH₂)ₙOCOR₂ or -(CH₂)ₙCOOR₂, the other represents a hydrogen atom; n represents an integer of 0 to 2; R₂ represents an alkyl or alkenyl group having 4 or more carbon atoms, Y₁ and Y₂ each independently represent any of a hydrogen atom, an alkyl group having 1 to 4 carbon atoms, a methoxy group, and a halogen atom, and r and p each independently represent an integer of 1 to 3.]

Among compounds represented by formula (1), those in which R₁ is a hydrogen atom are preferable, because a reversibly thermochromic composition with a wider hysteresis width is obtainable, and those in which R₁ is a hydrogen atom and m is 0 are more preferable.

Among the compounds represented by formula (1), more preferred are compounds expressed by the following formula (2). 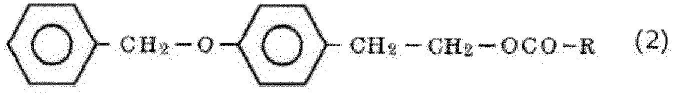 (wherein R is an alkyl or alkenyl group having 8 or more carbon atoms, preferably an alkyl group having 10 to 24 carbon atoms, and more preferably an alkyl group having 12 to 22 carbon atoms)

In addition, as the component (c), a compound expressed by the following formula (3) may be used. 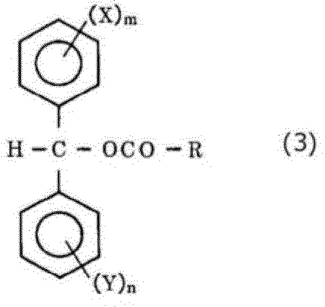 (wherein R represents an alkyl or alkenyl group having 8 or more carbon atoms, m and n each independently represent an integer of 1 to 3, and X and Y each independently represent any of a hydrogen atom, an alkyl group having 1 to 4 carbon atoms, an alkoxy group having 1 to 4 carbon atoms, and a halogen atom)

In addition, as the component (c), a compound expressed by the following formula (4) may be used. 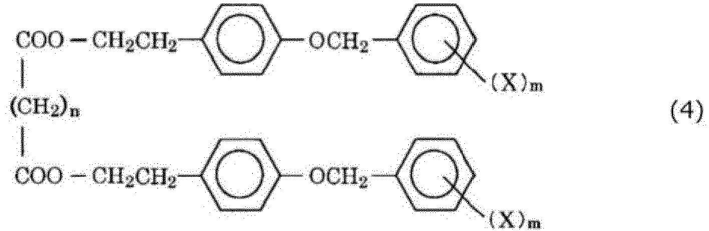 (wherein X represents a hydrogen atom, an alkyl group having 1 to 4 carbon atoms, a methoxy group, or a halogen atom, m represents an integer of 1 to 3, and n represents an integer of 1 to 20)

In addition, as the component (c), a compound expressed by the following formula (5) may be used. 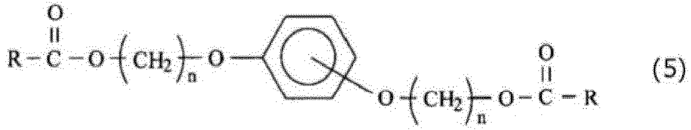 (wherein R represents an alkyl or alkenyl group having 1 to 21 carbon atoms, and n represents an integer of 1 to 3)

In addition, as the component (c), a compound expressed by the following formula (6) may be used. 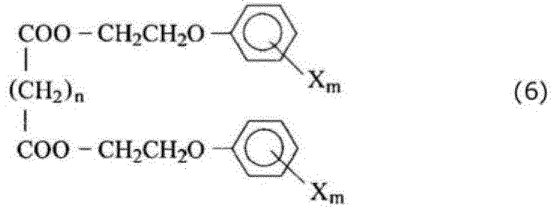 (wherein X represents a hydrogen atom, an alkyl group having 1 to 4 carbon atoms, an alkoxy group having 1 to 4 carbon atoms, or a halogen atom, m represents an integer of 1 to 3, and n represents an integer of 1 to 20)

In addition, as the component (c), a compound expressed by the following formula (7) may be used. 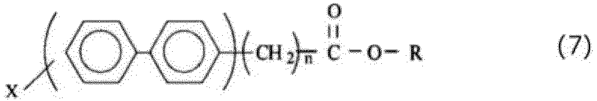 (wherein R represents an alkyl group having 4 to 22 carbon atoms, a cycloalkyl alkyl group, a cycloalkyl group, or an alkenyl group having 4 to 22 carbon atoms, X represents a hydrogen atom, an alkyl group having 1 to 4 carbon atoms, an alkoxy group having 1 to 4 carbon atoms, or a halogen atom, and n represents 0 or 1)

In addition, as the component (c), a compound expressed by the following formula (8) may be used. 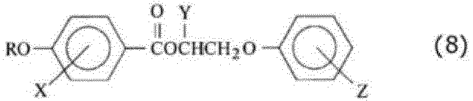 (wherein R represents an alkyl group having 3 to 18 carbon atoms or an aliphatic acyl group having 3 to 18 carbon atoms, X represents a hydrogen atom, an alkyl group having 1 to 3 carbon atoms, an alkoxy group having 1 or 2 carbon atoms, or a halogen atom, Y represents a hydrogen atom or a methyl group, and Z represents a hydrogen atom, an alkyl group having 1 to 4 carbon atoms, an alkoxy group having 1 or 2 carbon atoms, or a halogen atom)

In addition, as the component (c), a compound expressed by the following formula (9) may be used. 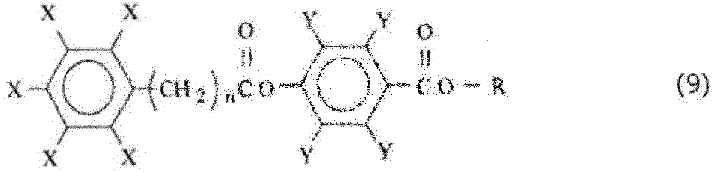 (wherein R represents an alkyl group having 4 to 22 carbon atoms, an alkenyl group having 4 to 22 carbon atoms, a cycloalkyl alkyl group, or a cycloalkyl group, X represents a hydrogen atom, an alkyl group, an alkoxy group, or a halogen atom, Y represents a hydrogen atom, an alkyl group, an alkoxy group or a halogen atom, and n represents 0 or 1)

In addition, as the component (c), a compound expressed by the following formula (10) may be used. 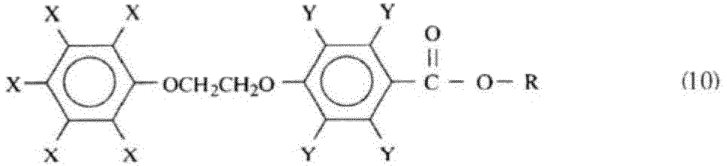 (wherein R represents an alkyl group having 3 to 18 carbon atoms, a cycloalkyl alkyl group having 6 to 11 carbon atoms, a cycloalkyl group having 5 to 7 carbon atoms, or an alkenyl group having 3 to 18 carbon atoms, X represents a hydrogen atom, an alkyl group having 1 to 4 carbon atoms, an alkoxy group having 1 to 3 carbon atoms, or a halogen atom, and Y represents a hydrogen atom, an alkyl group having 1 to 4 carbon atoms, a methoxy group, an ethoxy group or a halogen atom)

In addition, as the component (c), a compound expressed by the following formula (11) may be used. 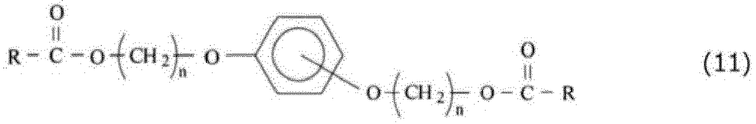 (wherein R represents a cycloalkyl group having 3 to 8 carbon atoms, or a cycloalkyl alkyl group having 4 to 9 carbon atoms, and n represents an integer of 1 to 3)

In addition, as the component (c), a compound expressed by the following formula (12) may be used. 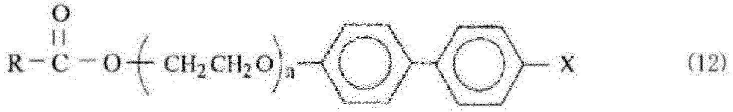 (wherein R represents an alkyl group having 3 to 17 carbon atoms, a cycloalkyl group having 3 to 8 carbon atoms, or a cycloalkyl alkyl group having 5 to 8 carbon atoms, X represents a hydrogen atom, an alkyl group having 1 to 5 carbon atoms, a methoxy group, an ethoxy group, or a halogen atom, and n represents an integer of 1 to 3)

Examples of the compounds represented by Formulas (2) to (12) include compounds described in JP 2020-100710 A.

Among the components (c), a compound having one or more aromatic rings is preferable because of excellent solubility of the component (a) in the reversibly thermochromic composition, and it becomes easy to obtain a reversibly thermochromic composition having an improved color development density. Among the compounds represented by Formulas (1) to (12), the compounds having two or more aromatic rings are more preferable.

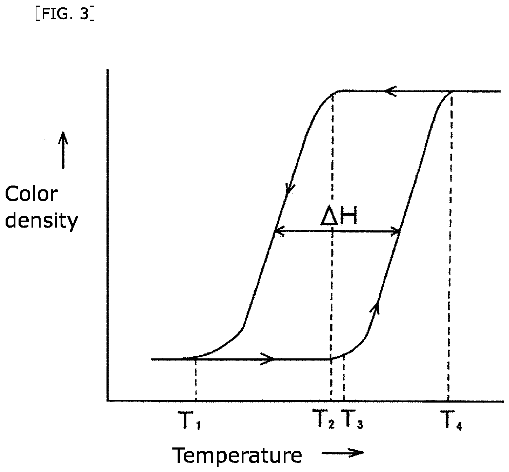

As an electron-accepting compound, a reversibly thermochromic composition of heat color-developing type (a color is developed by heating and lost by cooling) using a gallic ester (JP S51-44706 A, JP 2003-253149 A) or the like, and a reversibly thermochromic microcapsule pigment encapsulating the reversibly thermochromic composition can be applied (see FIG. 3).

The reversibly thermochromic composition according to the present invention is a compatible material containing, as essential components, the components (a), (b), and (c), and although the ratio of the components depends on the density, discoloration temperature, discoloration mode, and kind of each component, in general, a mass ratio of the component (a) : the component (b) at which desired characteristics are obtained is in a range of preferably 1 : 0.1 to 1 : 100, more preferably 1 : 0.1 to 1 : 50, and still more preferably 1 : 0.5 to 1 : 20.

A mass ratio of the component (a): the component (c) is in a range of preferably 1 : 5 to 1 : 100, more preferably 1 : 5 to 1 : 50, still more preferably 1 : 5 to 1 : 20, and particularly preferably 1 : 5 to 1 : 15. When the mass ratio of the component (a) and the component (c) is in the above range, it is easy to obtain a reversibly thermochromic composition having a large difference in density between the color density and the decolor density, that is, being more excellent in contrast between the colored state and the decolored state.

In addition, various light stabilizers may be blended in the reversibly thermochromic composition as necessary.

The light stabilizer is contained to prevent photodegradation of the reversibly thermochromic composition including the components (a), (b) and (c), and is blended in an amount of 0.3 to 24 parts by mass and preferably 0.3 to 16 parts by mass based on 1 part by mass of the component (a). Among the light stabilizers, an ultraviolet light absorber effectively cuts ultraviolet light contained in sunlight and the like to prevent a photo-deterioration that may be caused by excitation by the photo-reaction of the component (a). Antioxidants, singlet oxygen quenchers, superoxide anion quenchers, ozone quenchers, and the like restrain oxidation reaction due to light.

The light stabilizer can be used by appropriately mixing one kind or two or more kinds.

Although the reversibly thermochromic composition of the present invention is effective even when used as it is, the composition can be encapsulated in a microcapsule to form a reversibly thermochromic microcapsule pigment (hereinafter may be referred to as a "microcapsule pigment" or a "pigment"), or dispersed in a thermoplastic resin or a thermosetting resin to form reversibly thermochromic resin particles (hereinafter may be referred to as "resin particles").

The reversibly thermochromic composition is preferably encapsulated in a microcapsule to form a reversibly thermochromic microcapsule pigment. This is because a chemically and physically stable pigment can be constituted by encapsulating the reversibly thermochromic composition in a microcapsule, and, in addition, the reversibly thermochromic composition can maintain the same composition and the same working-effect can be obtained under various operation conditions.

Examples of the microencapsulation include conventionally known isocyanate-type interfacial polymerization, in situ polymerization using a melamine-formalin system or the like, submerged coat hardening method, phase separation from aqueous solution, phase separation from organic solvent, melt dispersion cooling method, aerial suspension coating method, and spray drying method. The microencapsulation is selected as needed, depending on the use purpose. Further, the microcapsule can be put into practical use after further forming a secondary resin coating film on the surface thereof in accordance with the intended use, so as to impart the microcapsule with durability or to modify the surface properties.

In the reversibly thermochromic microcapsule pigment, a mass ratio of the content and the membrane wall is preferably 7 : 1 to 1 : 1, and when the mass ratio of the content and the membrane wall is within the above range, a deterioration in the color density and vividness during color development is prevented. The mass ratio of the content and the membrane wall is more preferably 6 : 1 to 1 : 1.

An average particle diameter of the reversibly thermochromic microcapsule pigment or resin particles is preferably in a range of 0.01 to 50 µm, more preferably 0.1 to 30 µm, and still more preferably 0.5 to 20 µm. When the average particle diameter of the microcapsule pigment or the resin particles is more than 50 µm, the microcapsule pigment or the resin particles lack dispersion stability and processing suitability in blending into an ink, a paint, or a resin. On the other hand, when the average particle diameter is less than 0.01 µm, it is difficult to achieve a high-density color development.

When the microcapsule pigment or the resin particles are used in ink for writing instrument, the average particle diameter is in a range of preferably 0.01 to 5 µm, more preferably 0.05 to 4 µm, still more preferably 0.1 to 3 µm, and particularly preferably 0.5 to 3 µm. When the average particle diameter of the pigment or the resin particles is more than 5 µm, it is difficult to obtain good ink dischargeability when the pigment or the resin particles are used for a writing instrument. On the other hand, when the average particle diameter is less than 0.01 µm, it is difficult to achieve a high-density color development.

In the measurement of the average particle diameter, a region of particles is determined using an image analysis type particle size distribution measuring software [manufactured by Mountech Co., Ltd., product name: Mac-View], a projected area equivalent circle diameter (Heywood diameter) is calculated from the area of the region of particles, and the average particle diameter is measured as an average particle diameter of particles equivalent to an equal volume sphere based on the calculated value.

When the particle diameter of all particles or most of the particles exceed 0.2 µm, the average particle diameter can be measured as an average particle diameter of particles equivalent to an equal volume sphere by the Coulter method using a particle size distribution analyzer [manufactured by Beckman-Coulter, Inc., product name: Multisizer 4e].

In addition, a volume-based particle diameter and the average particle diameter may be measured by a laser diffraction/scattering-type particle size distribution analyzer [manufactured by Horiba, Ltd., product name: LA-300] after calibration based on the numerical values measured using the software or the analyzer by the Coulter method.

A reversibly thermochromic colorant, such as a reversibly thermochromic composition, a reversibly thermochromic microcapsule pigment, or resin particles is dispensed in a vehicle containing water and/or an organic solvent and as necessary various additives to be formed into an ink composition (hereinafter may be referred to as "ink"), so that the resulting ink composition can be used as a reversibly thermochromic liquid composition for: printing inks used in screen printing, offset printing, process printing, gravure printing, coater printing, pad printing, or the like; paints used in brush coating, spray coating, electrostatic coating, electrodeposition coating, flow coating, roller coating, dip coating, or the like; inks for ink jet use; UV curable inks; inks for writing instruments such as marking pens, ballpoint pens, fountain pens, and brush pens; inks for coating tools; inks for a stamp; painting colors; cosmetics; coloring liquids for fibers; and the like.

Various additives can be blended in the reversibly thermochromic liquid composition.

Examples of the additive include resins, cross-linking agents, curing agents, desiccants, plasticizers, viscosity-adjusting agents, dispersants, ultraviolet absorbers, antioxidants, light stabilizers, anti-settling agents, lubricants, gelling agents, antifoaming agents, delustering agents, penetrating agents, pH regulators, foaming agents, coupling agents, humectants, antifungal agents, preservatives, and anticorrosives.

As a vehicle for writing instrument used in ink for writing instrument, there may be mentioned an oily vehicle including an organic solvent, or an aqueous vehicle including water and if necessary an organic solvent.

When the vehicle is an aqueous vehicle, a water-soluble organic solvent compatible with water can be blended in the ink for writing instrument. The water-soluble organic solvent can suppress water evaporation of the ink, prevent fluctuations in the specific gravity of the vehicle to maintain good dispersion stability of the reversibly thermochromic microcapsule pigment, and stabilize a structure of a loose aggregate formed by a polymeric coagulant or the polymeric coagulant and a dispersant to be described later.

Examples of the organic solvent include ethanol, propanol, butanol, glycerin, sorbitol, triethanolamine, diethanolamine, monoethanolamine, ethylene glycol, diethylene glycol, thiodiethylene glycol, polyethylene glycol, propylene glycol, butylene glycol, ethylene glycol monomethyl ether, ethylene glycol monoethyl ether, ethylene glycol monobutyl ether, diethylene glycol monomethyl ether, diethylene glycol monoethyl ether, diethylene glycol monobutyl ether, propylene glycol monobutyl ether, ethylene glycol monomethyl ether acetate, sulfolane, 2-pyrrolidone, and N-methyl-2-pyrrolidone.

When the ink for writing instrument contains a water-soluble organic solvent, the water-soluble organic solvent is blended in a range of preferably 1 to 40% by mass, more preferably 5 to 30% by mass, and still more preferably 10 to 25% by mass based on a total amount of the ink. When the blending ratio of the water-soluble organic solvent exceeds 40% by mass, an ink viscosity is likely to increase. On the other hand, when the blending ratio is less than 1% by mass, the effect of suppressing moisture evaporation is poor.

When the ink for writing instrument contains the water-soluble organic solvent and the hysteresis width (ΔH) of the reversibly thermochromic microcapsule pigment blended in the ink for writing instrument is large, the specific gravity of the microcapsule pigment is larger than 1, and when the specific gravity of the vehicle is adjusted, it is easy to adjust the specific gravity if the water-soluble organic solvent having a specific gravity larger than that of water is used, so that it is preferable to use glycerin having a specific gravity exceeding 1.1 or the like as the water-soluble organic solvent.

A shear thinning imparting agent can be blended to the ink for writing instrument, and the ink containing the shear thinning imparting agent (shear thinning ink) can suppress cohesion and sinking of the microcapsule pigment, and can suppress spreading of the handwriting, so that a good handwriting can be formed.

In addition, when the shear thinning ink is stored in a writing instrument which is a ballpoint pen, it is possible to prevent a leakage of the ink from an interval between a ball and a tip when the writing instrument is not used, or to prevent a reverse flowing of the ink when a writing front-end is disposed upward (erect state).

Examples of the shear thinning imparting agent include xanthan gum, welan gum, succinoglycan (average molecular weight is about 1,000,000 to 8,000,000) that is an organic acid modified heteropolysaccharide of which constituent monosaccharides are glucose and galactose, alcagum, guar gum, locust bean gum and a derivative thereof, hydroxyethylcellulose, alkyl alginate esters, a polymer containing alkyl esters of methacrylic acid as a main component and having a molecular weight of 100,000 to 150,000, glucomannan, thickening polysaccharides having a gelation ability extracted from seaweeds such as agar or carrageenin, benzylidene sorbitol and benzylidene xylitol or a derivative of these, a crosslinkable acrylic acid polymer, inorganic fine particules, polyglycerine fatty acid ester, polyoxyethylene sorbitan fatty acid ester, polyethylene glycol fatty acid ester, polyoxyethylene castor oil, polyoxyethylene lanolin-lanolin alcohol-beeswax derivatives, polyoxyethylene alkyl ether-polyoxypropylene alkyl ether, polyoxyethylene alkyl phenyl ether, a non-ionic surfactant such as fatty acid amide having an HLB value of 8 to 12, salts of dialkyl or dialkenyl sulfosuccinate, a mixture of N-alkyl-2-pyrrolidone and an anionic surfactant, and a mixture of polyvinylalcohol and an acrylic resin.

The polymeric coagulant can be blended to the ink for writing instrument, and in the ink containing the polymeric coagulant (cohesive ink), the microcapsule pigment forms a loose aggregate through the polymeric coagulant, and the microcapsule pigments are prevented from coming in contact with each other and aggregating, so that dispersibility of the microcapsule pigment can be improved.

Examples of the polymeric coagulant include polyvinylpyrrolidones, polyethylene oxides and water-soluble polysaccharides.

Examples of the water-soluble polysaccharides include tragacanth gum, guar gum, pullulan, cyclodextrin, and an aqueous cellulose derivative.

In addition, examples of the aqueous cellulose derivatives include methyl cellulose, hydroxyethyl cellulose, hydroxypropyl cellulose, hydroxyethylmethyl cellulose, and hydroxypropylmethyl cellulose.

Among the polymeric coagulants, hydroxyethyl cellulose is preferable because of excellent dispersibility.

The polymeric coagulant is blended in a range of preferably 0.1 to 1% by mass, more preferably 0.3 to 0.5% by mass based on the total amount of the ink. When the content is in the above range, the microcapsule pigment forms a loose aggregate, and the effect of improving the dispersibility of the pigment can be sufficiently developed.

In addition, the dispersibility of the microcapsule pigment can be improved by blending a dispersant in the ink for writing instrument.

The polymeric coagulant and the dispersant can be used in combination. When both are used in combination, the dispersibility of the microcapsule pigment can be improved, and the dispersibility of the loose aggregate of the microcapsule pigment formed through the polymeric coagulant can be further improved.

Examples of the dispersant include synthetic resins such as polyvinylpyrrolidone, polyvinyl butyral, polyvinyl ether, styrene-maleic acid copolymers, ketone resins, hydroxyethyl cellulose and derivatives thereof, and styrene-acrylic acid copolymers; acrylic polymers; PO/EO adducts; and amine base oligomers of polyesters.

Among the above dispersants, an acrylic polymer dispersant is preferable, an acrylic polymer dispersant having a carboxy group is more preferable, and an acrylic polymer dispersant having a comb-like structure having a carboxy group in a side chain is still more preferable because of excellent dispersibility of the microcapsule pigment.

The dispersant is particularly preferably an acrylic polymer dispersant having a comb-like structure having a plurality of carboxy groups in side chains, and specific examples thereof include product name: Solsperse 43000 manufactured by Japan Lubrizol Corporation.

The dispersant is blended in a range of preferably 0.01 to 2% by mass, more preferably 0.1 to 1.5% by mass based on the total amount of the ink. When the blending ratio of the dispersant exceeds 2% by mass, the microcapsule pigment easily sinks or floats when vibration or the like is applied from the outside. On the other hand, when the blending ratio is less than 0.01% by mass, the effect of improving the dispersibility is hardly exhibited.

In addition, blending of an aqueous resin allows for imparting the ink for writing instrument with fixability on paper surface of the handwriting and viscosity.

Examples of the water-soluble resin include alkyd resins, acrylic resins, styrene-maleic acid copolymers, cellulose derivatives, polyvinylpyrrolidones, polyvinyl alcohols, and dextrin.

Among the water-soluble resins, polyvinyl alcohol is preferable because the acrylic polymer dispersant is excellent in stability, and in addition, a partial saponification type polyvinyl alcohol having a degree of saponification of 70 to 89% by mol is more preferable because it has good solubility even when the ink is in an acidic range.

The water-soluble resin is blended in a range of preferably 0.3 to 3.0% by mass, more preferably 0.5 to 1.5% by mass based on the total amount of the ink.

In addition, when viscosity of the vehicle used in the ink for writing instrument is low, by blending a specific gravity adjuster, it is possible to prevent the microcapsule pigment from sinking or floating, and localizing in the ink and to improve the dispersion stability of the microcapsule pigment.

The dispersion stability of the pigment is maximized when a difference in specific gravity between the vehicle and the pigment is minimal, and the specific gravity adjuster brings the specific gravity of the vehicle closer to the specific gravity of the pigment. Since the specific gravity of the vehicle depends on the specific gravity of an aqueous substance dissolved in the vehicle and the amount thereof added, when a more amount of the specific gravity adjuster having a large specific gravity is added and dissolved in the vehicle, the specific gravity of the vehicle can be further increased.

Examples of the specific gravity adjuster include an oxyacid belonging to Group 6 in the range of atomic weight 90 to 185 and salt thereof.

Such a specific gravity adjuster can adjust the specific gravity of the vehicle such that the vehicle approaches a pigment having a large specific gravity, and can prevent the pigment from sinking or floating, and localizing due to application of a stimulus such as vibration from the outside while the ink has a low viscosity.

The oxyacid and salt thereof are selected from the group consisting of oxyacid of a transition metal element and salt thereof, and it is said that the oxyacid ion is obtained by forming a tetrahedron or octahedron in which four or six oxygen atoms are usually coordinated to metal atoms or the like.

The oxyacid and the salt thereof may be a polyacid and a polyacid salt which is a salt of the polyacid, the polyacid includes an isopoly acid, a heteropoly acid, and the like, and the polyacid salt includes an isopoly acid salt, a heteropoly acid salt, and the like.

Examples of the specific gravity adjuster include single oxygen acid and salt thereof, isopoly acid and salt thereof, and heteropoly acid and salt thereof.

Examples of the single oxygen acid include molybdic acid and tungstic acid, and examples of the salt of the single oxygen acid include sodium molybdate, potassium molybdate, ammonium molybdate, sodium tungstate, potassium tungstate, ammonium tungstate, lithium tungstate, and magnesium tungstate.

Examples of the isopoly acid include metamolybdic acid, paramolybdic acid, metatungstic acid, paratungstic acid, and isotungstic acid, and examples of the isopoly acid salt include sodium metamolybdate, potassium metamolybdate, ammonium metamolybdate, sodium paramolybdate, potassium paramolybdate, ammonium paramolybdate, sodium metatungstate, potassium metatungstate, ammonium metatungstate, barium metatungstate, sodium paratungstate, and sodium isotungstate.

Examples of the heteropoly acid include molybdophosphoric acid, molybdosilicic acid, tungstophosphoric acid, and tungstosilicic acid, and examples of the heteropoly acid salt include sodium molybdophosphate, sodium molybdosilicate, sodium tungstophosphate, and sodium tungstosilicate.

The above-mentioned oxyacid and salt thereof can be used by appropriately mixing one kind or two or more kinds.

Among the specific gravity adjusters described above, there are preferred metatungstic acid, paratungstic acid, sodium metatungstate, potassium metatungstate, ammonium metatungstate, barium metatungstate, sodium paratungstate, sodium isotungstate, tungstophosphoric acid, tungstosilicic acid, sodium tungstophosphate, and sodium tungstosilicate, and more preferred are sodium isotungstate, sodium metatungstate and sodium paratungstate.

The above-mentioned sodium isotungstate, sodium metatungstate, and sodium paratungstate are not only highly safe, but also have a high specific gravity per se, so that it is easy to adjust a liquid with a high specific gravity according to the amount added, and they are suitably used.

The specific gravity adjuster is blended in a range of preferably 2 to 20% by mass, more preferably 5 to 15% by mass based on the total amount of the ink. When the blending ratio of the specific gravity adjuster exceeds 20% by mass, the microcapsule pigment is likely to aggregate. On the other hand, when the blending ratio is less than 2% by mass, the effect of adjusting the specific gravity of the vehicle is poor.

The mass ratio of the microcapsule pigment and the specific gravity adjuster is preferably 1: 0.05 to 4.0, more preferably 1: 0.075 to 2.0, and still more preferably 1: 0.1 to 1.5.

The vehicle containing the specific gravity adjuster is particularly effective for a microcapsule pigment having a large specific gravity, can suppress sinking of the pigment in the ink when a stimulus such as vibration is externally applied while the ink has a low viscosity, and can improve the dispersion stability of the microcapsule pigment.

Although the specific gravity of the microcapsule pigment depends on the particle diameter, components encapsulated in the microcapsule and their contents, the component and membrane thickness of the wall membrane of the microcapsule, and the colored state and temperature of the microcapsule pigment, the specific gravity is preferably in a range of 1.05 to 1.20 when the microcapsule pigment is in a completely colored state and water is used as a reference at 20°C. Although such a pigment exhibits a property of having a large hysteresis width (ΔH), decolors by heating, and can maintain a decolored state in a specific temperature range, a pigment having a large hysteresis width (ΔH) tends to have a large specific gravity because a compound having two or more aromatic rings in the molecule is often used as the component (c), and tends to sink and separate in the ink, and in particular, when a stimulus such as vibration is applied from the outside, the pigment tends to sink and separate. However, in the ink containing the specific gravity adjuster, sinking and localization of the microcapsule pigment are suppressed while the ink has a low viscosity, and the dispersion stability of the pigment can be improved; therefore, the ink is suitably used.

In consideration of the dispersion stability of the pigment in the ink, the specific gravity of the microcapsule pigment is preferably 1.10 to 1.20 and more preferably 1.12 to 1.15 when the microcapsule pigment is in the completely colored state and water is used as a reference at 20°C.

The specific gravity of the microcapsule pigment can be measured by the following method.

### (Method of measuring specific gravity of microcapsule pigment)

1. 30 ml of an aqueous glycerin solution and 1 g of a completely colored state microcapsule pigment are introduced into a screw tube bottle and mixed to obtain a microcapsule pigment dispersion.
2. The temperature of 30 ml of the microcapsule pigment dispersion is adjusted to 20°C, and the microcapsule pigment dispersion is set in a centrifuge at a rotation speed of 1000 rpm for 30 seconds. As the centrifuge, a desktop cooling centrifuge [manufactured by Kokusan Co., Ltd., product name: H103N] can be used.
3. The microcapsule pigment dispersion is observed.

When most of the microcapsule pigment is precipitated at a bottom of a beaker, the operations of 1 to 2 are performed again using an aqueous solution having a higher glycerin concentration than an aqueous glycerin solution at this time, and the state of the dispersion is observed.

When a state where most of the microcapsule pigment are floating on the liquid surface is confirmed, the operations of 1 to 2 are performed again using an aqueous solution having a lower glycerin concentration than then aqueous glycerin solution at this time, and the state of the dispersion is observed.

The above series of operations are repeated until not the state where most of the microcapsule pigments are floating on the liquid surface or are precipitated, but a state where the portions other than the liquid surface of the aqueous glycerin solution and the vicinity of the bottom of the screw tube bottle are uniformly colored is visually confirmed. The specific gravity of the aqueous glycerin solution at the time when this state is observed is measured, and the measured specific gravity is determined as the specific gravity of the microcapsule pigment. The specific gravity of the glycerin aqueous solution can be measured by the buoy method described in JIS K0061 7.1 for an aqueous solution adjusted to 20°C.

The vehicle containing the specific gravity adjuster has a specific gravity in a range of 1.00 to 1.30 when water is used as a reference material at 20°C, and the specific gravity is preferably 1.05 to 1.20 and more preferably 1.08 to 1.18.

In addition, the specific gravity of the vehicle is preferably 0.90 to 1.20 times and more preferably 0.95 to 1.10 times the specific gravity of the pigment.

When the specific gravity of the vehicle is in the above range and the specific gravity of the vehicle with respect to the specific gravity of the pigment is in the above range, in a case where the stimulus such as vibration is externally applied to the ink, it is possible to further suppress sinking and localization of the pigment in the ink are suppressed while the ink has a low viscosity, and to further improve the dispersion stability of the pigment.

When the vehicle for writing instrument is an aqueous vehicle, the vehicle contains at least water, and the water is blended in a range of preferably 30 to 80% by mass and more preferably 40 to 70% by mass based on the total amount of the ink.

Moreover, when the ink for writing instruments is used in a ballpoint pen, it is preferable that the abrasion of a ball receiving sheet be prevented by adding, to the ink, lubricants including higher fatty acids such as oleic acid, non-ionic surfactants having a long chain alkyl group, polyether modified silicone oil, thiophosphorous acid triesters such as thiophosphorous acid tri(alkoxycarbonyl methyl ester) or thiophosphorous acid tri(alkoxycarbonyl ethyl ester), phosphoric acid monoester of polyoxyethylene alkyl ether or polyoxyethylene alkylaryl ether, phosphoric acid diester of polyoxyethylene alkyl ether or polyoxyethylene alkylaryl ether, and metal salts, ammonium salts, amine salts, and alkanolamine salts thereof.

In addition, additives such as a wetting agent, a resin, resin particles, a pH adjusting agent, a rust inhibitor, a surfactant, a humectant, an antifoaming agent, a viscosity adjusting agent, an antiseptic agent, and an antifungal agent can be blended as necessary.

The reversibly thermochromic microcapsule pigment is blended in the ink for writing instrument in an amount of preferably 5 to 40% by mass, more preferably 10 to 40% by mass, and still more preferably 10 to 30% by mass based on the total amount of the ink. When the blending ratio of the microcapsule pigment is in the above range, a desirable color development density can be obtained, and deterioration of the ink outflow properties can be inhibited.

The ink composition according to the present invention can be produced by any conventionally known method. Specifically, the above components are added in required amounts and mixed with various agitators such as a propeller stirrer, a homodisper, or a homomixer, or various dispersers such as a bead mill, whereby the ink composition can be produced.

When the ink for writing instrument according to the present invention is used in a ballpoint pen, the viscosity of the ink is preferably 1 to 2000 mPa·s, more preferably 3 to 1500 mPa·s, and still more preferably 500 to 1000 mPa·s when the viscosity is measured under the condition of a rotation speed of 1 rpm (sear rate of 3.84 sec⁻¹) in an environment of 20°C since sinking or aggregation of the microcapsule pigment can be suppressed. Furthermore, the viscosity is preferably 1 to 200 mPa·s, more preferably 10 to 100 mPa·s, and still more preferably 20 to 50 mPa·s when the viscosity is measured under the condition of a rotation speed of 100 rpm (sear rate of 384 sec⁻¹) in an environment of 20°C since ink dischargeability from a pen tip of the ballpoint pen can be improved. When the viscosity is in the above range, the dispersion stability of the microcapsule pigment and easy fluidity of the ink in a mechanism of the ballpoint pen can be maintained at a high level.

The viscosity is a value measured using a rheometer [manufactured by TA Instruments, product name: Discovery HR-2, cone plate (diameter of 40 mm, angle of 1°)] under the condition of a rotation speed of 1 rpm (shear rate of 3.84 sec⁻¹) or a rotation speed of 100 rpm (shear rate of 384 sec⁻¹) with the ink placed in an environment of 20°C.

When the ink for writing instrument according to the present invention is used in a ballpoint pen, a surface tension of the ink is preferably 20 to 50 mN/m, and more preferably 25 to 45 mN/m in an environment of 20°C. When the surface tension is in the above range, it is easy to suppress spreading of the handwriting and striking-through a sheet of paper, and the wettability of the ink to the sheet of paper can be improved.

The surface tension is a value measured with a surface tension measuring instrument [manufactured by Kyowa Interface Science Co., Ltd., product name: DY-300] by a vertical plate method using a platinum plate with the ink placed in an environment of 20°C.

When the ink for writing instrument according to the present invention is used in a ballpoint pen, a pH of the ink is preferably 3 to 10, and more preferably 4 to 9. When the pH is in the above range, aggregation or sinking of the microcapsule pigment contained in the ink in a low temperature range can be suppressed.

The pH is a value measured with a pH meter [manufactured by DKK-TOA CORPORATION, product name: IM-40S type] with the ink placed in an environment of 20°C.

When the ink for writing instrument according to the present invention is used in a marking pen, the viscosity of the ink is preferably 1 to 20 mPa·s, more preferably 1 to 10 mPa·s, and still more preferably 1 to 5 mPa·s when the viscosity is measured under the condition of a rotation speed of 30 rpm in an environment of 20°C. When the viscosity is in the above range, fluidity of the ink and the dispersion stability of the microcapsule pigment can be improved.

The viscosity is a value measured using a BL-type rotational viscometer [manufactured by Toki Sangyo Co., Ltd., product name: TVB-M-type viscometer, L-type rotor] with the ink placed in an environment of 20°C.

When the ink for writing instrument according to the present invention is used in a marking pen, the surface tension of the ink is preferably 25 to 50 mN/m, more preferably 25 to 45 mN/m, and still more preferably 35 to 45 mN/m in an environment of 20°C. When the surface tension is in the above range, it is easy to suppress spreading of the handwriting and striking-through a sheet of paper, and the wettability of the ink to the sheet of paper can be improved.

The surface tension is a value measured with a surface tension measuring instrument [manufactured by Kyowa Interface Science Co., Ltd., product name: DY-300] by the vertical plate method using a glass plate with the ink placed in an environment of 20°C.

When the ink for writing instrument according to the present invention is used in a marking pen, the pH of the ink is preferably 3 to 8, more preferably 4 to 7, and still more preferably 5 to 6. When the pH is in the above range, aggregation or sinking of the microcapsule pigment contained in the ink in a low temperature range can be suppressed.

The pH is a value measured with a pH meter [manufactured by DKK-TOA CORPORATION, product name: IM-40S type] with the ink placed in an environment of 20°C.

The ink for writing instrument is stored in a writing instrument including a pen tip and an ink filling mechanism.

Examples of the writing instrument include various writing instruments such as a ballpoint pen, a marking pen, a fountain pen, a brush pen, and a calligraphy pen.

The pen tip of the writing instrument is not particularly limited, and a pen tip including various tips is used.

Among the various tips, examples of a ballpoint pen tip include a tip formed by holding a ball in a ball holding part in which the vicinity of a front-end of a metal pipe is pressed and deformed inwardly from the outside; a tip formed by holding a ball in a ball holding part formed by cutting a metal material by a drill and the like; a tip in which a ball receiving sheet made of a resin is provided in the tip made of metal or plastic; and a tip in which a ball held in the tip is pressed in a front direction by a spring.

The material of the ballpoint pen tip and the ball is not particularly limited, and examples thereof include cemented carbide, stainless steel, ruby, ceramic, resin, and rubber.

The diameter of the ball is preferably 0.1 to 3.0 mm, more preferably 0.2 to 2.0 mm, and still more preferably 0.3 to 1.0 mm. The ball can also be subjected to surface treatment such as DLC coating.

Examples of the marking pen tip include a generally known porous member having communication pores of which porosity is selected within a range of about 30 to 70%, made of processed resin of fibers, fusion processed bodies of hot-melt fiber, a felt, or the like, and an extrusion molded product of a synthetic resin having a plurality of ink delivering holes extending in the axial direction, and the tip is provided for practical use by processing its one end in a cannonball form, a rectangular form, or a chisel form depending on the purpose.

Examples of a tip (pen body) of a fountain pen shape include one obtained by cutting a metal plate such as a stainless steel plate or a gold alloy plate to a tapered form, followed by bending or curving, and one obtained by resin molding to a pen tip form. A slit can be provided at the center of the pen body, or a bead portion can also be provided at an end thereof.

Examples of the ink filling mechanism include an ink storage body and an ink occlusion body that can be directly filled with the ink for writing instrument.

As the ink storage body, for example, a molded article made of a thermoplastic resin such as polyethylene, polypropylene, polyethylene terephthalate, or nylon or a metal tube is used, and the ink storage body and the tip may be connected via a connection member in addition to directly connecting the tips.

The ink occlusion body is a fiber bundle in which crimped fibers are bundled in a length direction, and is configured by incorporating into a covering material such as a plastic tube or a film, and adjusting its porosity within a range of about 40 to 90%.

When the ink for writing instrument is filled in a ballpoint pen, the structure and shape of the ballpoint pen itself are not particularly limited. Examples thereof include a ballpoint pen having an axial barrel body in which a shear thinning ink is filled in an ink storage, in which the ink storage body is connected with a ballpoint pen tip where a ball is mounted on its front-end, and a liquid plug for preventing backflow is closely contacted in the edge of the ink.

An ink follower composition is filled at the rear end of the ink filled in the ink storage body.

The ink follower composition includes a non-volatile liquid and/or a hardly volatile liquid, and examples thereof include vaseline, spindle oil, castor oil, olive oil, mineral oil refineries, liquid paraffine, polybutene, α-olefine, oligomer and co-oligomer of α-olefine, dimethyl silicone oil, methylphenyl silicone oil, amino modified silicone oil, polyether modified silicone oil, and fatty acid modified silicone oil.

The ink follower composition can be used by appropriately using one kind or two or more kinds in combination.

The ink follower composition is preferably thickened to a suitable viscosity by adding a thickening agent.

Examples of the thickening agent include silica having hydrophobic treated surface; particulate silica having a methylated surface; aluminum silicate; swellable mica; a clay-based thickening agent such as hydrophobically treated bentonite or montmorilonite; fatty acid metal soaps such as magnesium stearate, calcium stearate, aluminum stearate, and zinc stearate; a dextrin-based compound such as tribenzylidene sorbitol, fatty acid amide, amide modified polyethylene wax, hydrogenated castor oil, or fatty acid dextrin; and a cellulose-based compound.

In addition, the liquid ink follower composition and a solid ink follower composition can be used in combination.

The axial barrel itself can be used as an ink filling mechanism, and a ballpoint pen in which ink is directly filled in the axial barrel and a ballpoint pen tip is attached to a front end of the axial barrel can be exemplified.

The ballpoint pen including the ballpoint pen tip and the ink filling mechanism may further include an ink supply mechanism for supplying the ink to be filled in the ink filling mechanism to the pen tip.

The ink supply mechanism is not particularly limited, and examples thereof include (1) a mechanism including an ink guide core formed from a fiber bundle or the like as an ink flow rate regulator and supplying ink to a pen tip, (2) a mechanism including a comb-groove-shaped ink flow rate regulator and supplying the ink to the pen tip with the ink flow regulator interposed therebetween, and (3) a mechanism in which a large number of disk bodies are arranged in parallel at intervals in a comb groove shape, a slit-shaped ink guide groove longitudinally penetrating the disk body in the axial direction and a ventilation groove having a larger width than the groove are provided, and the ink is supplied to the pen tip through a pen core in which an ink guide core for guiding the ink from the ink filling mechanism to the pen tip is disposed at an axial center.

The material of the pen core is not particularly limited as long as it is a synthetic resin capable of injection-molding a large number of disk bodies into a comb-groove-shaped structure. Examples of the synthetic resin include general-purpose polycarbonate, polypropylene, polyethylene, and an acrylonitrile-butadiene-styrene copolymer (ABS resin). In particular, an acrylonitrile-butadiene-styrene copolymer (ABS resin) is suitably used because the acrylonitrile-butadiene-styrene copolymer has high moldability and can easily obtain pen core performance.

When the ballpoint pen includes the above-mentioned ink supply mechanism, the above-mentioned ink occlusion body can also be used as the ink filling mechanism in addition to the ink storage body and the axial barrel described above.

Specific examples of the configuration of the ballpoint pen that contains the ink for writing instrument include (1) a ballpoint pen in which a ballpoint pen tip is connected to an ink storage body directly or via a connection member, the ink for writing instrument is filled, and a ballpoint pen refill formed by filling an ink follower on an end surface of the ink is contained in an axial barrel, (2) a ballpoint pen in which the ink for writing instrument is directly filled in the axial barrel, and a mechanism for supplying the ink to the pen tip by interposing an ink guide core, including a comb-groove-shaped ink flow rate regulator, a fiber bundle, and the like, as an ink flow rate regulator is provided, (3) a ballpoint pen in which the ink for writing instrument is directly filled in the axial barrel, and a mechanism for supplying the ink to the pen tip via the above-mentioned pen core is provided, and (4) a ballpoint pen in which an ink occlusion body formed from a fiber bundle impregnated with ink for writing instrument is contained in the axial barrel, and a mechanism for supplying the ink to the pen tip by interposing an ink guide core, formed from a fiber bundle and the like, as an ink flow rate regulator is provided.

When the ink for writing instrument is filled in the marking pen, the structure and shape of the marking pen itself are not particularly limited, and examples thereof include a marking pen which has an axial barrel body in which an ink occlusion is filled with a cohesive ink and in which the ink occlusion body communicates with the marking pen tip.

The ink occlusion body may be directly connected with the tip, or the ink occlusion body may be connected with the tip via a connection member.

The marking pen including a marking tip and the ink filling mechanism may further include the ink supply mechanism for supplying the ink to be filled in the ink filling mechanism to the pen tip.

The ink supply mechanism is not particularly limited, and examples thereof include, in addition to the ink supply mechanism included in the ballpoint pen described above, (4) a mechanism that includes an ink flow rate regulator by a valve mechanism and supplies the ink to the pen tip by valve opening.

As the valve mechanism, a generally known pumping type which is opened by pressing a tip can be used, and a valve mechanism which is set to a spring pressure which can be opened by pressing force by writing pressure is preferable.

When the marking pen includes the ink supply mechanism, an ink storage body that can be directly filled with the ink for writing instrument can be used as the ink filling mechanism in addition to the above-mentioned ink occlusion body. The axial barrel itself may be used as the ink filling mechanism to directly fill the ink for writing instrument.

Specific examples of the configuration of the marking pen that contains the ink for writing instrument include (1) a marking pen in which an ink occlusion body formed from a fiber bundle impregnated with the ink for writing instrument is contained in an axial barrel, and a marking pen tip formed from a fiber processed body or a resin molded body having a capillary gap formed is connected to the axial barrel directly or via a connection member such that the ink occlusion body and the tip are connected to each other, (2) a marking pen in which a ballpoint pen in which the ink for writing instrument is directly filled in the axial barrel, and a mechanism for supplying the ink to the pen tip by interposing an ink guide core, including a comb-groove-shaped ink flow rate regulator, a fiber bundle, and the like, as an ink flow rate regulator is provide, (3) a marking pen in which the ink for writing instrument is directly filled in the axial barrel, and a mechanism for supplying the ink to the pen tip via the above-mentioned pen core is provided, (4) a marking pen which is provided with a tip and an ink storage body through a valve mechanism for opening a valve by pressing the tip and in which the ink for writing instrument is directly filled in the ink storage body, and (5) a marking pen containing, in the axial barrel, a marking pen refill in which the marking pen tip formed from the fiber processed body or the resin molded body having the capillary gap formed is connected to an ink storage body containing the ink occlusion body formed from a fiber bundle impregnated with ink for writing instrument directly or via the connection member such that the ink occlusion body and the tip are connected to each other.

In addition, the ballpoint pen or the marking pen described above can be in the form of an ink cartridge as a detachable structure. In this case, after the ink contained in an ink cartridge of the writing instrument is used up, the writing instrument can be used by being replaced with a new cartridge.

As the ink cartridge, an ink cartridge that also serves as the axial barrel constituting the writing instrument by being connected to a writing instrument main body, or an ink cartridge that covers and protects the axial barrel (rear shaft) after being connected to the writing instrument main body is used. In the latter case, the ink cartridge may be used alone, and, in addition, an ink cartridge in which the writing instrument main body and the ink cartridge are connected in the writing instrument before use, or an ink cartridge contained in the axial barrel in a non-connected state so that a user of the writing instrument starts using the writing instrument by connecting the ink cartridge in the axial barrel at the time of use may be used.

When the ballpoint pen or the marking pen is directly filled with the ink for writing instrument, in order to facilitate redispersion of the microcapsule pigment, it is preferable to incorporate a stirring body such as a stirring ball for stirring the ink in the ink storage body or the axial barrel filled with the ink. Examples of the shape of the stirring body include a spherical body and a rod body. The material of the stirring body is not particularly limited, and examples thereof include metal, ceramic, resin, and glass.

In addition, it is preferable that the writing instrument such as a ballpoint pen or a marking pen is provided with a cap attached so as to cover a writing distal end portion (tip distal end portion) or an in-and-out type mechanism that allows the writing distal end portion to be retractable from the writing instrument main body (axial barrel), and it is possible to prevent the writing distal end portion from being dried and incapable of writing and to prevent the writing distal end portion from being contaminated or damaged.

Any writing instrument provided with the in-and-out type mechanism can be used as long as the writing instrument is stored in the axial barrel in a state where the writing distal end portion is exposed to the outside air, and the writing distal end portion protrudes from an opening of the axial barrel by the operation of the in-and-out type mechanism. For example, the above-mentioned ballpoint pen refill or marking pen refill is produced, the refill is stored in the axial barrel, and the writing distal end portion protrudes from the axial barrel opening by the operation of the in-and-out type mechanism, so that a writing instrument provided with the in-and-out type mechanism (retractable writing instrument) can be produced.

When the in-and-out type mechanism is provided in the writing instrument, a composite retractable writing instrument (retractable ballpoint pen or retractable marking pen) in which a plurality of ballpoint pen refills or marking pen refills are stored in the axial barrel and the writing distal end portion of any of the refills is retracted from the axial barrel opening by the operation of the in-and-out type mechanism can be provided.

Examples of the in-and-out type mechanism include (1) a side slide type in-and-out type mechanism in which an operation portion (clip) movable in a front-rear direction from a rear side wall of an axial barrel is projectingly provided outward in a radial direction, and a writing front-end is ejected from and put in through a fore-ended opening portion of the axial barrel by sliding the operation portion forward, (2) a rear end knock type in-and-out type mechanism in which an operation portion provided at a rear end of an axial barrel is pressed forward, whereby a writing front-end is ejected from and put in through a fore-ended opening part of the axial barrel, (3) a side knock type in-and-out type mechanism in which an operation portion protruding from an outer surface on the axial barrel side is pressed inward in a radial direction, whereby a writing front-end is ejected from and put in through a fore-ended opening part of the axial barrel, and (4) a rotation type in-and-out type mechanism in which a writing front-end is ejected from and put in through a fore-ended opening part of the axial barrel by rotating and operating an operation portion of a rear part of the axial barrel.

In addition, the form of the ballpoint pen or marking pen is not limited to the above configuration, a multiple writing instrument (such as a both head type or a pen-front drawing type) in which tips of different type may be attached, or pen fronts for introducing inks of different colors may be attached, and, in addition, tips of different type are attached and color tones of inks delivered from the respective tips are different may be used.

The handwriting obtained by carrying out writing on a writing surface using a writing instrument containing the ink for writing instruments can change the color by rubbing with a finger and a heating tool or a cooling tool.

As the heating tool, an electro-heating discoloration device equipped with a resistance heating element such as a PTC element, a heating discoloration device loaded with a medium such as hot water, a heating discoloration device using steam or laser light, or a hair dryer can be used, and a friction member and a frictional body are preferable because discoloration can be achieved by a simple method.

As the cooling tool, an electro-cryogenic discoloration device using a Peltier element, a cryogenic discoloration device loaded with a refrigerant such as cold water or crushed ice, a refrigerant, a refrigerator, or a freezer can be used.

As the friction member and the frictional body, an elastic body such as an elastomer or a plastic foamed body, which has a good elasticity and can generate adequate friction at the time of scratching and generate frictional heat is preferable; however, a plastic molded body, stone, wood, metal, cloth, and the like may be used as well.

Although the handwriting may be rubbed with an ordinary rubber eraser used for erasing handwriting with a pencil, since eraser crumbs are generated during the rubbing, the friction member and the frictional body which hardly generate crumbs are preferably used.

Examples of the material of the friction member and the frictional body include a silicone resin, an SEBS resin (styrene-ethylene-butadiene-styrene block copolymer). In the silicone resin, the resin is likely to attach to a portion erased by friction, and a handwriting tends to be repelled after writing in repetition; therefore, the SEBS resin is more preferably used.

The friction member or the frictional body may be a member having an arbitrary shape separate from the writing instrument, and excellent portability can be achieved when the friction member or the frictional body is provided in the writing instrument. A writing instrument set can also be obtained by combining the writing instrument with a friction member or a frictional body having an arbitrary shape separate from the writing instrument.

In the case of a writing instrument equipped with a cap, the position at which the friction member or the frictional body is provided is not particularly restricted. For example, the cap itself can be formed by the friction member; the axial barrel itself can be formed by the friction member; when a clip is arranged, the clip itself can be formed by the friction member; or the friction member or the frictional body can be provided on the front end of the cap (crown) or the rear end of the axial barrel (the part where a writing front-end is not arranged).

When the writing instrument is the retractable writing instrument, the position at which the friction member or the frictional body is provided is not particularly restricted. For example, the axial barrel itself can be formed by the friction member; when a clip is further provided, the clip itself can be formed by the friction member; or the friction member or the frictional body can be provided in the vicinity of an opening of the axial barrel, on the rear end of the axial barrel (the part where a writing front-end is not arranged), or on the knocking part.

The ink described above can also be used as an ink for a stamp.

Although water is used as a medium of the ink for a stamp, a water-soluble organic solvent can also be used as necessary.

When the microcapsule pigment is used for the ink for a stamp, among the water-soluble organic solvents, glycerin or propylene glycol is preferable.

The water-soluble organic solvent is blended in a range of preferably 30 to 60% by mass, more preferably 30 to 55% by mass, still more preferably 40 to 50% by mass based on the total amount of the ink. When the blending ratio of the water-soluble organic solvent is in the above range, the ink is not dried or absorbed, and a clear stamp image is easily obtained.

When the blending ratio of the water-soluble organic solvent exceeds 60% by mass, hygroscopicity tends to be high, and a stamp image is blurred, or spots appear, so that it is difficult to obtain a clear stamp image. On the other hand, when the blending ratio is less than 30% by mass, a stamp surface is dried, the stamp image becomes faint, and it is difficult to obtain a clear stamp image.

An organic solvent can also be used as the medium described above.

Examples of the organic solvent include castor oil fatty acid alkyl esters, cellosolve solvents, alkylene glycol solvents, ester solvents, hydrocarbon solvents, halogenated hydrocarbon solvents, alcohol solvents, ether solvents, ketone solvents, propionic acid solvents, highly polar solvents, and mixed solvents thereof.

In addition, a thickening agent may be blended in the ink for a stamp. Among the thickening agents, an alkali-soluble acrylic emulsion is preferable.

When the alkali-soluble acrylic emulsion is used as the thickening agent, the pH of the ink is preferably 6 to 11, more preferably 7 to 11, and still more preferably 7 to 10.

In addition, by adding a binder resin to the ink for a stamp, fixability of a stamp image can be enhanced, and the viscosity of the ink can be adjusted.

Examples of the binder resin include a resin emulsion, an alkali-soluble resin, and a water-soluble resin.

In addition, additives such as a wetting agent, a resin, resin particles, a pH adjusting agent, a rust inhibitor, a surfactant, a humectant, an antifoaming agent, a viscosity adjusting agent, an antiseptic agent, and an antifungal agent can be blended as necessary.

The reversibly thermochromic microcapsule pigment is blended in the ink for a stamp in an amount of preferably 10 to 40% by mass, more preferably 10 to 35% by mass, and still more preferably 10 to 30% by mass based on the total amount of the ink. When the blending ratio of the microcapsule pigment exceeds 40% by mass, the dispersion stability of the microcapsule pigment in the ink tends to be deteriorated. On the other hand, when the blending ratio is less than 10% by mass, the color development density tends to be deteriorated.

The ink for a stamp described above can be used as an ink for a stamp pad, and an ink for a stamp provided with a stamp material having continuous pores.

For example, a stamp pad for supplying an ink to a stamp face of a stamp to be contacted can be obtained by impregnating the ink into a stamp pad. Further, a stamp can be obtained by impregnating an ink into a stamp material having continuous pores mounted on a stamp.

The stamp can form a stamp image on various surfaces to be stamped. In addition, the stamp image formed by the ink for a stamp can be made to change a color by rubbing with a finger or the application of the heating tool or the cooling tool described above. The friction member and the frictional body described above is preferable as the heating tool because color changing can be achieved by a simple method.

The friction member or the frictional body may be a member having an arbitrary shape separate from the stamp, and excellent portability can be achieved when the friction member or the frictional body is provided in the stamp. A stamp set can also be obtained by combining the stamp with a friction member or a frictional body having an arbitrary shape separate from the stamp.

The material of the support is not particularly limited when the reversibly thermochromic liquid composition is applied or printed, and every material is effective. Examples thereof include paper, synthetic paper, fiber, fabric, synthetic leather, leather, plastics, glass, pottery materials, metals, wood, and stone.

The shape of the support is not limited to a flat shape and may be an irregular form.

A reversibly thermochromic laminate (reversibly thermochromic printed matter) can be obtained by providing a reversibly thermochromic layer containing a reversibly thermochromic colorant on a support.

In cases where a non-thermochromic colored layer (non-thermochromic image) has been formed on the support in advance, the colored layer or an image can be made visible or invisible depending on temperature change by applying thereto a reversibly thermochromic layer, and this enables to further diversify the mode of change.

In addition, the reversibly thermochromic colorant is melt blended with an excipient and molded to obtain a reversibly thermochromic solid molded article for coating, which can be used as a solid writing material or a solid cosmetic material.

Examples of the solid writing material include crayons, pencil leads, mechanical pencil leads, and solid gel markers.

Examples of the solid cosmetic material include foundation creams, eyeliners, eyebrow paints, eye shadows, and lipsticks.

Examples of the excipient used for the solid writing material include waxes, gelation agents, and clay minerals.

Among the excipients, at least one of a polyolefin wax, a sucrose fatty acid ester, and a dextrin fatty acid ester is preferably contained from the viewpoint of easily improving the writing density.

The excipient preferably has a mass average molecular weight (Mw) of 2,000 to 50,000, and more preferably has a weight average molecular weight of 10,000 to 30,000, from the viewpoint of excellent mechanical strength and thermochromic characteristics of the solid writing material and easy handling at the time of production. The excipient preferably has a number average molecular weight (Mn) of 1,000 to 10,000.

The mass average molecular weight and the number average molecular weight are values measured by gel permeation chromatography (GPC) based on polystyrene.

The excipient is blended in a range of preferably 0.2 to 70% by mass, more preferably 0.5 to 40% by mass based on the total amount of the solid writing material. When the blending ratio of the excipient is in the above range, the shape as the solid writing material is easily obtained, and the writing density of the solid writing material is easily increased.

When the blending ratio of the excipient exceeds 70% by mass, it is difficult to obtain a sufficient writing density. On the other hand, when the blending ratio is less than 0.2% by mass, it is difficult to obtain a shape as a writable core material.

By blending a filler in the solid writing material, the strength of the solid writing material can be improved, and writing feel can be adjusted.

Among the fillers, talc or calcium carbonate is preferable because it is excellent in moldability and hardly impairs the thermochromic characteristics when a microcapsule pigment is used.

The filler is blended in a range of preferably 10 to 65% by mass based on the total amount of the solid writing material. When the blending ratio of the filler exceeds 65% by mass, color developability and writing feel tend to be deteriorated. On the other hand, when the blending ratio is less than 10% by mass, the strength of the solid writing material tends to be deteriorated.

In addition, the strength of the solid writing material can be improved by blending a binder resin in the solid writing material.

Among the binder resins, an ethylene-vinyl acetate copolymer, an ethylene-vinyl alcohol copolymer, and a polyvinyl alcohol are preferable, and by using these resins in combination with a polyester polyol, molding stability can be improved.

The binder resin is blended in a range of preferably 0.5 to 5% by mass based on the total amount of the solid writing material.

By blending a hindered amine compound in the solid writing material, it is possible to make it difficult to visually recognize a residual image of a portion where the handwriting of the writing surface is erased. Thus, re-writability can be satisfied without impairing appearance of the writing surface, and merchantability can be enhanced.

In addition, additives such as a viscosity-adjusting agent, an antifungal agent, a preservative, an antibacterial agent, an ultraviolet inhibitor, an antioxidant, a lubricant, and a perfume can be blended as necessary.

The solid writing material may be used alone as a writing material, or may have a core-sheath structure (double core) provided with an outer shell covering an outer peripheral surface thereof using the solid writing material as an inner core.

An additive such as a non-thermochromic colorant, an antifungal agent, a preservative, an antibacterial agent, an ultraviolet absorber, an antioxidant, a lubricant, and a perfume can be blended in the outer shell as necessary.

The solid writing material is capable of carrying out writing on various writing surfaces, and in addition, the reversibly thermochromic colorant is used; therefore, the handwriting obtained by writing on the writing surface can be made to change a color by rubbing with a finger or the application of the heating tool or the cooling tool described above. The friction member and the frictional body described above is preferable as the heating tool because color changing can be achieved by a simple method.

Although the friction member or the frictional body described above may be a member having an arbitrary shape separate from a solid writing material or an exterior of the solid writing instrument housing the solid writing material in an exterior housing object, excellent portability is achieved by providing the friction member or the frictional body in the solid writing material or the exterior of the solid writing instrument housing the solid writing material in the exterior housing object. Specifically, for example, this may be in the form in which a friction member is provided on a wooden or paper external packaging shaped like a pencil, a crayon, etc. A solid writing material set can also be obtained by combining the solid writing material with a friction member or a frictional body having an arbitrary shape separate from the solid writing material.

In addition, the reversibly thermochromic colorant is melt blended with a thermoplastic resin, a thermosetting resin, waxes, or the like to form a pellet, a powder, or a paste, and can be used as a resin composition for forming a reversibly thermochromic molded article.

From the resin composition for forming a reversibly thermochromic molded article, three dimensional shaped body having a predetermined shape, and moldings such as films, sheets, plates, filaments, rods or pipes, or the like are obtained by a conventional means such as injection molding, extrusion molding, blow molding or cast molding.

A toner and a powder coating may be obtained by melt-blending into the thermoplastic resin.

A non-thermochromic colorant, such as a generally employed dye or pigment, may be added to the above-mentioned reversibly thermochromic liquid composition, solid molded article for coating, or molding resin to cause color change behavior from color (1) to color (2).

Light resistance can be improved by stacking a layer containing a light stabilizer and/or transparent metalescent pigment or durability can be improved by providing a topcoat layer thereon over the molded article or the laminate described above.

Examples of the light stabilizer include ultraviolet absorbers, antioxidants, singlet oxygen quenchers, superoxide anion quenchers, and ozone quenchers.

Examples of the transparent metallic luster pigment include pigments prepared by coating the surface of a core substance, such as natural mica, synthetic mica, glass piece, alumina, or transparent film piece, with a metal oxide such as titanium oxide.

Specific examples of products using the reversibly thermochromic composition and the microcapsule pigment encapsulating the same or the resin particles are listed below.
(1) Toys:
   dolls and animal-figured toys; hair of dolls and animal-figured toys; dollhouses and furniture thereof; doll accessories such as clothes, hats, bags, and shoes; accessory toys; stuffed dolls and animals; painting toys; illustrated books for toys; puzzle toys such as jigsaw puzzles; toy bricks; block toys; clay toys; fluid toys; spinning tops; kites; musical toys; cooking toys; gun toys; capturing toys; background toys; toys imitating vehicles, animals, plants, buildings, and food articles; and the like
(2) Clothing:
   outerwears such as T-shirts, sweaters, blouses, dresses, swimsuits, raincoats, and ski wears; footwears such as shoes and shoelaces; personal effects made of cloth, such as handkerchiefs, towels, and wrapping cloths; gloves; neckties; hats; scarves; mufflers; and the like
(3) Interior Ornaments:
   curtains, curtain cords, tablecloth, matting, cushions, carpets, rugs, chair upholstery, seats, mats, picture frames, imitation flowers, photo stands, and the like
(4) Furniture:
   beddings such as bedclothes, pillows, and mattresses; lighting fixtures; air conditioners; and the like
(5) Accessories:
   rings, bracelets, tiaras, earrings, hair stoppers, artificial nails, ribbons, scarfs, watches, glasses, and the like
(6) Stationeries:
   writing instruments, stamps, erasers, celluloid boards, rulers, notebooks, adhesive tapes, and the like
(7) Daily Necessaries:
   cosmetics such as lipsticks, eye-shadows, foundation creams, eyeliners, eyebrow paints, manicures, hair dyes, artificial nails, and paints for artificial nails; toothbrushes; and the like
(8) Kitchen Utensils:
   cups, dishes, chopsticks, spoons, forks, pots, frying pans, and the like
(9) Other Products:
   various printed articles, such as calendars, labels, cards, recording materials, and those for forgery prevention; books such as illustrated books; bags; packaging containers; embroidery threads; sporting gears; fishing gears; coasters; musical instruments; pocket warmers; refrigerants; pouches such as wallets; umbrellas; vehicles; buildings; indicators for temperature detection; training and learning articles; and the like.

### [Examples]

Examples will be described below. Unless otherwise specified, "part(s)" and "%" in the examples indicate "part(s) by mass" and "% by mass", respectively.

### <Example 101>

### Preparation of reversibly thermochromic microcapsule pigment

A reversibly thermochromic composition reversibly changing its color was obtained by mixing 6.5 parts of 2-(3-trifluoromethylanilino)-6-di-n-pentylaminofluorane (A-1) as the component (a), 4 parts of 4,4'-[1-{4-[1-(4-hydroxyphenyl)-1-methylethyl]phenyl}ethylidene]bisphenol (I-1) and 6 parts of 1,1-bis(4-hydroxyphenyl)-2-ethylhexane (IIa-1) as the components (b), and 25 parts of behenyl alcohol (C-1) and 25 parts of stearyl stearate (C-2) as the components (c), followed by heating to dissolve them. The reversibly thermochromic composition was charged into a mixed solution composed of 35 parts of an aromatic isocyanate prepolymer as a wall membrane material and 40 parts of an auxiliary solvent, and then emulsified and dispersed in a 8% polyvinyl alcohol aqueous solution. Stirring was continued while heating, then 2.5 parts of a water-soluble aliphatic-modified amine was added, and stirring was further continued to prepare a microcapsule dispersion. A microcapsule pigment having an average particle diameter of 2.0 µm was obtained from the microcapsule dispersion by a centrifugal separation method.

### <Examples 102 to 106 and 201 to 209 and Comparative Examples 101, 102, and 201 to 209>

A microcapsule pigment was obtained in the same manner as in Example 101 except that the kinds and addition amounts of the components (a), (b), and (c) were changed to those described in Table 1 or 2.

The color tone of the colored state of the obtained microcapsule pigment was as described in Tables 1 and 2, and the colored state was changed to the decolored state.

The numerical values of the components (a), (b), and (c) in the table indicate "part(s) by mass", and the numerical value of a concentration retention rate indicates "%".

| Table 1 | | | Example | | | | | | Comparative Example | |
|---|---|---|---|---|---|---|---|---|---|---|
| | | | 101 | 102 | 103 | 104 | 105 | 106 | 101 | 102 |
| Compo sition | (a) | A-1 | 6.5 | 6.5 | 6.5 | 6.5 | 6.5 | 6.5 | 6.5 | 6.5 |
| | (b) | I-1 | 4 | 4 | 5 | 5 | 5 | 5 | 10 | 10 |
| | | IIa-1 | 6 | 6 | | | | | | |
| | | IIa-2 | | | 5 | | | | | |
| | | IIa-3 | | | | 5 | | | | |
| | | IIa-4 | | | | | 5 | | | |
| | | IIb-1 | | | | | | 5 | | |
| | (c) | C-1 | 25 | | | | | | 25 | |
| | | C-2 | 25 | | | | | | 25 | |
| | | C-3 | | 50 | 50 | 50 | 50 | 50 | | 50 |
| Color tone | | | Black | Black | Black | Black | Black | Black | Black | Black |
| Evalua tion | Color development density | | 1.78 | 1.75 | 1.83 | 1.84 | 1.90 | 1.69 | 1.72 | 1.70 |
| | Decoloring density | | 0.04 | 0.04 | 0.05 | 0.06 | 0.07 | 0.09 | 0.04 | 0.04 |
| | Concentration retention rate | | 95.5 | 96.6 | 96.2 | 96.7 | 94.7 | 94.1 | 90.1 | 89.4 |

| Table 2 | | | Exam ple | Com parat ive Exam ple | Exam pie | Com parat ive Exam ple | Exam pie | Com parat ive Exam ple | Exam pie | Com parat ive Exam ple | Exam pie | Com parat ive Exam ple | Exam pie | Com parat ive Exam ple | Exam ple | Com parat ive Exam ple | Exam ple | Com parat ive Exam ple | Exam ple | Com parat ive Exam ple |
|---|---|---|---|---|---|---|---|---|---|---|---|---|---|---|---|---|---|---|---|---|
| | | | 201 | 201 | 202 | 202 | 203 | 203 | 204 | 204 | 205 | 205 | 206 | 206 | 207 | 207 | 208 | 208 | 209 | 209 |
| Com positi on | (a) | A-2 | 5 | 5 | | | | | | | | | | | | | 5 | 5 | | |
| | | A-3 | | | 1 | 1 | | | | | | | | | | | | | | |
| | | A-4 | | | | | 6 | 6 | | | | | | | | | | | | |
| | | A-5 | | | | | | | 6 | 6 | | | | | | | | | | |
| | | A-6 | | | | | | | | | 2 | 2 | | | | | | | | |
| | | A-7 | | | | | | | | | | | 2.75 | 2.75 | | | | | | |
| | | A-8 | | | | | | | | | | | | | 1.5 | 1.5 | | | | |
| | | A-9 | | | | | | | | | | | | | | | | | 2.5 | 2.5 |
| | (b) | 1-1 | 4 | 10 | 3 | 8 | 4 | 12 | 4 | 12 | 4 | 10 | 4 | 10 | | | | | | |
| | | 1-2 | | | | | | | | | | | | | 5 | 10 | | | | |
| | | 1-3 | | | | | | | | | | | | | | | 5 | 10 | | |
| | | 1-4 | | | | | | | | | | | | | | | | | 5 | 10 |
| | | IIa-1 | 6 | | 5 | | 8 | | 8 | | 6 | | 6 | | 5 | | 5 | | 5 | |
| | (c) | C-3 | 50 | 50 | | | 50 | 50 | 50 | 50 | 50 | 50 | | | 50 | 50 | | | 50 | 50 |
| | | C-4 | | | 50 | 50 | | | | | | | 50 | 50 | | | 50 | 50 | | |
| Color tone | | | Red | Red | Pink | Pink | Oran ge | Oran ge | Green | Green | Blue-gree n | Blue-gree n | Blue | Blue | Blue | Blue | Red | Red | Blue-gree n | Blue-gree n |
| Evalu ation | Color developmen | | 2.39 | 2.24 | 1.47 | 1.43 | 1.52 | 1.40 | 1.83 | 1.68 | 2.23 | 2.05 | 2.05 | 2.00 | 1.90 | 1.72 | 2.24 | 2.21 | 2.37 | 2.16 |
| | Decoloring density | | 0.10 | 0.13 | 0.03 | 0.04 | 0.09 | 0.10 | 0.01 | 0.02 | 0.06 | 0.07 | 0.03 | 0.04 | 0.02 | 0.03 | 0.09 | 0.09 | 0.08 | 0.07 |
| | Concentrati on retention | | 93.3 | 85.3 | 97.3 | 92.3 | 93.4 | 84.3 | 94.5 | 85.7 | 97.3 | 93.2 | 58.5 | 57.5 | 98.9 | 97.7 | 90.2 | 76.0 | 95.8 | 90.3 |

The components (a), (b), and (c) in the table are compounds shown below.

| | |
|---|---|
| A-1 | 2-(3-trifluoromethylanilino)-6-di-n-pentylaminofluorane |
| A-2 | 6-(N-ethyl-N-isopentylamino)-1,2-benzofluorane |
| A-3 | 2-di-n-butylamino-8-di-n-pentylamino-4-methylspiro[5H-[1]benzopyrano[2,3-d]pyrimidine-5,1'(3'H)isobenzofuran]-3'-one |
| A-4 | 1,3-dimethyl-6-diethylaminofluoran |
| A-5 | 2-N-methylanilino-6-(N-ethyl-N-p-tolylamino)fluoran |
| A-6 | 3,3-bis(2-ethoxy-4-diethylaminophenyl)-4-azaphthalide |
| A-7 | 3,3-bis(4-dimethylaminophenyl)-6-dimethylaminophthalide, |
| A-8 | 3,6-bis(diphenylamino)fluoran, |
| A-9 | 3-(2-acetamide-4-diethylaminophenyl)-3-(1-propyl-2-methylindole-3-yl)-4-azaphthalide, |
| I-1 | 4,4'-[1-{4-[1-(4-hydroxyphenyl)-1-methylethyl]phenyl}ethylidene] bisphenol |
| I-2 | 4,4',4"-ethylidinetrisphenol, |
| I-3 | 1-phenyl-1,1-bis(4-hydroxyphenyl)ethane |
| I-4 | 4,4'-[4-(4-hydroxyphenyl)-sec-butylidene]bis(2-methylphenol). |

| | |
|---|---|
| IIa-1 | 1,1-bis(4-hydroxyphenyl)-2-ethylhexane |
| IIa-2 | 2,2-bis(4-hydroxyphenyl)propane |
| IIa-3 | 1,1-bis(4-hydroxyphenyl)-2-methylpropane |
| IIa-4 | 2,2-bis(4-hydroxyphenyl)hexafluoropropane |
| IIb-1 | 2,4-dihydroxy-4'-tert-butylbenzophenone |
| C-1 | behenyl alcohol |
| C-2 | stearyl stearate |
| C-3 | 4-benzyloxyphenylethyl caprate |
| C-4 | 4-biphenyl cyclohexylmethyl acetate |

### [Discoloration temperature measurement]

40 parts of each of the microcapsule pigments obtained in Examples 101 to 106 and 201 to 209 and Comparative Examples 101, 102, and 201 to 209, 52 parts of an ethylene-vinyl acetate copolymer resin emulsion, 5 parts of a thickening agent, and 3 parts of a leveling agent were mixed to prepare a reversibly thermochromic ink. A sample for measuring discoloration temperature was obtained by screen-printing a solid pattern on a high-quality paper by using the ink. Each of the samples for measuring discoloration temperature was placed in a measurement portion of a colorimeter (manufactured by Tokyo Denshoku, Co., Ltd., product name: TC-3600), temperature of the sample portion was increasing or decreasing at a speed of 2°C/min to measure a brightness value as color density at each temperature, to thereby plot a color density-temperature curve. From the color density-temperature curve, the complete coloring temperature t₁, the coloring starting temperature t₂, the decoloring starting temperature t₃, the complete decoloring temperature t₄, and ΔH [hysteresis width: (temperature at a midpoint between t₃ and t₄) - (temperature at a midpoint between t₁ and t₂)] were determined. The obtained results are as described in Tables 3 and 4 below.

The numerical value in the table indicates "°C".

| Table 3 | Example | | | | | | Comparative Example | |
|---|---|---|---|---|---|---|---|---|
| | 101 | 102 | 103 | 104 | 105 | 106 | 101 | 102 |
| t₁ | 48 | -20 | -20 | -20 | -20 | -20 | 50 | -20 |
| t₂ | 54 | -17 | -16 | -16 | -14 | -14 | 54 | -16 |
| t₃ | 53 | 40 | 42 | 42 | 42 | 40 | 54 | 40 |
| t₄ | 58 | 60 | 58 | 58 | 58 | 59 | 59 | 60 |
| ΔH | 4.5 | 68.5 | 68.0 | 68.0 | 67.0 | 66.5 | 4.5 | 68.0 |

| Table 4 | Exam pie | Comp arativ e Exam ple | Exam ple | Comp arativ e Exam ple | Exam ple | Comp arativ e Exam ple | Exam ple | Comp arativ e Exam ple | Exam ple | Comp arativ e Exam ple | Exam ple | Comp arativ e Exam ple | Exam ple | Comp arativ e Exam ple | Exam ple | Comp arativ e Exam ple | Exam ple | Comp arativ e Exam ple |
|---|---|---|---|---|---|---|---|---|---|---|---|---|---|---|---|---|---|---|
| | 201 | 201 | 202 | 202 | 203 | 203 | 204 | 204 | 205 | 205 | 206 | 206 | 207 | 207 | 208 | 208 | 209 | 209 |
| t₁ | -20 | -20 | -20 | -20 | -20 | -20 | -20 | -20 | -20 | -20 | -20 | -20 | -20 | -20 | -20 | -20 | -20 | -20 |
| t₂ | -15 | -14 | -13 | -6 | -15 | -8 | -16 | -10 | -10 | -12 | -15 | -10 | -6 | -5 | -15 | -17 | -15 | -15 |
| t₃ | 40 | 42 | 38 | 41 | 40 | 40 | 34 | 42 | 39 | 42 | 42 | 50 | 42 | 51 | 39 | 39 | 35 | 39 |
| t₄ | 59 | 59 | 61 | 60 | 60 | 59 | 59 | 59 | 60 | 60 | 59 | 60 | 60 | 61 | 58 | 58 | 59 | 60 |
| ΔH | 67.0 | 67.5 | 66.0 | 63.5 | 67.5 | 63.5 | 64.5 | 65.5 | 64.5 | 67.0 | 68.0 | 70.0 | 64.0 | 68.5 | 66.0 | 67.0 | 64.5 | 67.0 |

### [Density measurement]

40 parts of each of the microcapsule pigments obtained in Examples 101 to 106 and 201 to 209 and Comparative Examples 101, 102, and 201 to 209, 52 parts of an ethylene-vinyl acetate copolymer resin emulsion, 5 parts of a thickening agent, and 3 parts of a leveling agent were mixed to prepare a reversibly thermochromic ink. A sample for density measurement was obtained by screen-printing a solid pattern on a high-quality paper by using the ink.

Each of the samples for density measurement of Examples 101 to 106 and 201 to 209 and Comparative Examples 101, 102, and 201 to 209 cooled to t₁ or lower and brought into the completely colored state was set in the measurement portion of the fluorescent spectrodensitometer [manufactured by Konica Minolta, Inc., product name: FD-7], and an absolute density of the colored state (hereinafter, referred to as "color development density") was measured.

Each of the samples for density measurement of Examples 101 to 106 and 201 to 209 and Comparative Examples 101, 102, and 201 to 209 heated to t₄ or higher and brought into a completely decolored state was set in the measurement portion of the fluorescent spectrodensitometer described above, and an absolute density of the decolored state (hereinafter, referred to as "decoloring density") was measured. The obtained results are as described in Tables 1 and 2.

### [Light resistance evaluation]

40 parts of each of the microcapsule pigments of Examples 101 to 106 and 201 to 209 and Comparative Examples 101, 102, and 201 to 209, 52 parts of an ethylene-vinyl acetate copolymer resin emulsion, 5 parts of a thickening agent, and 3 parts of a leveling agent were mixed to prepare a reversibly thermochromic ink. A sample for measurement was obtained by screen-printing a solid pattern on a high-quality paper by using the ink.

Each of the above samples for measurement was cooled to t₁ or lower and brought into the completely colored state, and then set in a measurement portion of a fluorescent spectrodensitometer [manufactured by Konica Minolta, Inc., product name: FD-7], and an absolute density (hereinafter, referred to as "initial density") of the completely colored state was measured.

Next, each measurement sample whose density was measured was continuously irradiated with light for 10 hours at an irradiance of 170 w/m² using a xenon light resistance tester [manufactured by Suga Test Instruments Co., Ltd., product name: Table Sun XT 75] under a temperature environment not exceeding t₃.

Each sample for measurement after light irradiation was taken out, cooled to t₁ or lower, and brought into the completely colored state, then each sample for measurement was set in the measurement portion of the fluorescent spectrodensitometer, and the absolute density of the completely colored state after light irradiation (hereinafter, the absolute density is referred to as "density after light irradiation") was measured.

From a value of the initial density and a value of the density after light irradiation, the density retention rate [(value of density after light irradiation)/(value of initial density) × 100] was determined. The larger the numerical value of the density retention rate, the better the light resistance. The obtained results are as described in Tables 1 and 2.

### Application Example 1

### Preparation of reversibly thermochromic writing instrument (reversibly thermochromic ballpoint pen)

A reversibly thermochromic liquid composition as an ink for writing instrument was prepared by mixing 25 parts of the microcapsule pigment of Example 102 (cooled to -20°C or lower in advance to develop black), 0.3 parts of a shear thinning imparting agent (xanthan gum), 10 parts of urea, 10 parts of glycerin, 0.5 parts of a nonionic permeability imparting agent [manufactured by San Nopco Limited, product name: Nopco SW-WET-366], 0.1 parts of a modified silicone antifoaming agent [manufactured by San Nopco Limited, product name: Nopco 8034], 0.5 parts of a phosphoric acid ester surfactant [manufactured by DKS Co., Ltd., product name: PLYSURF AL], 0.5 parts of a pH regulator (triethanolamine), 0.2 parts of an antifungal agent [manufactured by Lonza Japan, product name: Proxel XL-2], and 52.9 parts of water.

The ink for writing instruments described above was sucked and filled in an ink-storing tube made of a polypropylene pipe and then connected, via a holder made of a resin, with a ballpoint pen tip holding a cemented carbide ball having a diameter of 0.5 mm on its front end. Then, a viscoelastic ink follower (liquid plug) containing polybutene as a main component was filled from the rear end of the ink-storing tube to produce a ballpoint pen refill. This refill was incorporated into an axial barrel to obtain a ballpoint pen (retractable ballpoint pen).

The ballpoint pen had a structure in which the ballpoint pen refill was stored in the axial barrel with a tip provided thereon being exposed to the outside air and the tip was projected from the front-end opening of the axial barrel by the action of a clip-shaped in-and-out type mechanism (sliding mechanism) arranged on a rear side wall of the axial barrel. The rear end portion of the axial barrel has an SEBS resin attached as a friction member.

When a black letter (handwriting) was formed by writing on a sheet of paper using the ballpoint pen described above, the handwriting showed black at room temperature (25°C), and the letter discolored and became colorless when the letter was rubbed using the friction member. This state could be maintained as long as the sheet of paper was not cooled to a temperature of -20°C or lower. Meanwhile, when the sheet of paper was put in a freezer and cooled to -20°C or lower, the letter showed the color change behavior of turning black again, and the color change behavior was repeatedly reproducible.

### Application Example 2

### Production of reversibly thermochromic recording material (information display card)

40 parts of the microcapsule pigment of Example 105 (cooled to -20°C or lower in advance to develop black) was uniformly mixed in an aqueous vehicle composed of 50 parts of a urethane resin emulsion, 3 parts of a leveling agent, and 1 part of a thickening agent to prepare a reversibly thermochromic liquid composition as a printing ink.

A transparent anchor coat layer composed of a urethane resin and an isocyanate-based curing agent was provided on a surface of a transparent polyester film (thickness: 25 µm) having an adhesive layer on a back surface as a support, and the printing ink described above was solidly printed on an upper layer of the anchor coat layer using a screen printing plate, and dried and cured to provide a reversibly thermochromic layer. In addition, a transparent protective layer containing an epoxy acrylate oligomer, a polyester acrylate oligomer, and an acrylate monomer was provided on an upper layer thereof, irradiated with ultraviolet rays, and polymerized to produce a reversibly thermochromic recording material. Then, the recording material was adhered to a white polyester film (thickness: 188 µm) as a base material, and put to practical use as an information display card.

The reversibly thermochromic recording material was once cooled to - 20°C or lower, and the reversibly thermochromic layer completely developed black. Thereafter, letter information was printed with a thermal printer having a thermal head.

In the recording material, white letter information (open-faced letter) was clearly displayed on a black background, and the white letter information was visually recognized as long as the recording material was held in a temperature range of more than -20°C and less than 58°C. When the recording material was cooled to -20°C or lower and the reversibly thermochromic layer completely developed black, the white open-faced letter was not visually recognized. From this state, it was possible to form a white open-faced letter on the reversibly thermochromic layer again using a thermal printer, and the recording material could be repeatedly used many times.

### Application Example 3

### Production of reversibly thermochromic printed matter

A reversibly thermochromic liquid composition as an ink for offset printing was prepared by mixing 30 parts of the microcapsule pigment of Example 106 (cooled to -20°C or lower in advance to develop black), 5 parts of a red dye, and 65 parts of a linseed oil-based offset ink vehicle.

Offset printing was performed on both front and back surfaces of high quality paper as a printing medium using the ink for offset printing, and drying and curing were performed to form a date (thermochromic image). The thermochromic images of the front surface and the back surface are formed so as not to overlap each other. Next, offset printing was performed using a non-discoloring ink for black offset printing, and drying and curing were performed to form a frame line (non-discoloring image), thereby producing a reversibly thermochromic printed matter.

Although the reversibly thermochromic printed matter described above was a printed matter in the form of a notebook in which a black date was formed at an initial stage, the color could be changed to red by frictional heat generated by rubbing the thermochromic image at an arbitrary position on the surface using the friction member, and the discolored state could be maintained at room temperature (25°C), so that the reversibly thermochromic printed matter was useful for schedule management of holidays. Since the date provided on the back surface of the discolored portion did not change its colordue to heat transfer when the color of the thermochromic image on the front surface was changed, accurate schedule management could be performed.

### Application Example 4

### Production of toy figure with hair using reversibly thermochromic composite fiber

5 parts of the microcapsule pigment of Example 201, 1 part of a dispersant, nylon 12 (94 parts) having a melting point of 180°C, and 0.1 parts of a general blue pigment were melt-mixed at 200°C with an extruder to prepare a resin composition for forming a reversibly thermochromic molded article in a pellet form for a core part.

The pellet described above was supplied to an extruder for forming a core, and a nylon 12 natural pellet was supplied to an extruder for forming a sheath. Using a composite fiber spinning apparatus, spinning was performed at 200°C from an 18-hole discharge port so that a volume ratio of the core : the sheath was 6 : 4, to prepare reversibly thermochromic composite fibers consisting of 18 single yarns having an outer diameter of 90 µm.

When the reversibly thermochromic composite fiber was temporarily cooled to -20°C or lower to completely develop the color of the microcapsule pigment, the reversibly thermochromic composite fiber showed purple in which red due to the microcapsule pigment and blue due to the general pigment were mixed. The reversibly thermochromic composite fiber was transplanted to a doll head by a conventional method to produce a toy figure with hair using the reversibly thermochromic composite fiber.

The hair of the toy figure did not change with body temperature and environmental temperature, and turned from purple to blue when heated to 59°C or higher. When cooled to -20°C or lower, the hair was changed to purple again. This change could be repeated.

It was possible to arbitrarily change the color of the hair by decoloring a part of the hair by heating with a dryer or the like, to form a pattern in which only an arbitrary portion was decolored. The discolored state could be maintained at room temperature (25°C), and by heating the whole hair to 59°C or higher to decolor and then cooling the hair to -20°C or lower, it was possible to allow the hair to develop purple again.

### Application Example 5

### Preparation of reversibly thermochromic writing instrument (reversibly thermochromic marking pen)

A reversibly thermochromic liquid composition as an ink for writing instrument was prepared by mixing 20 parts of the microcapsule pigment of Example 202 (cooled to -20°C or lower in advance to develop pink) in an aqueous vehicle composed of 0.4 parts of a polymeric coagulant (hydroxyethyl cellulose) [manufactured by Dow Chemical Japan Co., Ltd., product name: CELLOSIZE EP-09], 0.4 parts of an acrylic polymer dispersant [manufactured by Japan Lubrizol Corporation, product name: Solsperse 43000], 0.2 parts of a preservative (sodium 2-pyridinethiol-1-oxide) [manufactured by Lonza Japan, product name: Sodium Omadine], 0.2 parts of a preservative (3-iodo-2-propynyl N-butylcarbamate) [manufactured by Lonza Japan, product name: Glycacil 2000], 18 parts of glycerin, 0.2 parts of an antifoaming agent, 1 part of a pH regulator (10% diluted phosphoric acid solution), 8 parts of a specific gravity adjuster (sodium polytungstate) (manufactured by SOMETU, product name: SPT), and 46.6 parts of water.

An ink occlusion body prepared by covering a polyester sliver with a synthetic resin film was impregnated with the ink for writing instrument and contained in an axial barrel made of a polypropylene resin, the axial barrel was assembled with a resin-processed pen body (cannonball shape) formed of an extrusion molded product of a polyacetal resin having a plurality of ink delivering holes extending in the axial direction via a holder in such a manner that the front end of the axial barrel was in connection with the pen body, and a cap was fitted thereto to prepare a marking pen. The crown of the cap has an SEBS resin attached as a friction member.

When a pink letter (handwriting) was formed by writing on a sheet of paper using the marking pen described above, the handwriting showed pink at room temperature (25°C), and the letter discolored and became colorless when the letter was rubbed using the friction member. This state could be maintained as long as the sheet of paper was not cooled to a temperature of -20°C or lower. Meanwhile, when the sheet of paper was put in a freezer and cooled to -20°C or lower, the letter showed the color change behavior of turning pink again, and the color change behavior was repeatedly reproducible.

### Application Example 6

### Preparation of reversibly thermochromic writing instrument (reversibly thermochromic marking pen)

A reversibly thermochromic liquid composition as an ink for writing instrument was prepared by mixing 23 parts of the microcapsule pigment of Example 203 (cooled to -20°C or lower in advance to develop orange) in an aqueous vehicle composed of 0.4 parts of a polymeric coagulant (hydroxyethyl cellulose) [manufactured by Dow Chemical Japan Co., Ltd., product name: CELLOSIZE WP-09], 0.4 parts of an acrylic polymer dispersant [manufactured by Japan Lubrizol Corporation, product name: Solsperse 43000], 0.2 parts of a preservative (sodium 2-pyridinethiol-1-oxide) [manufactured by Lonza Japan, product name: Sodium Omadine], 0.2 parts of a preservative (3-iodo-2-propynyl N-butylcarbamate) [manufactured by Lonza Japan, product name: Glycacil 2000], 30 parts of glycerin, 0.01 parts of an antifoaming agent, 0.03 parts of a pH regulator (10% diluted phosphoric acid solution), and 45.76 parts of water.

An ink occlusion body prepared by covering a polyester sliver with a synthetic resin film was impregnated with the ink for writing instruments described above and inserted into an axial barrel made of a polypropylene resin. Then, the axial barrel was assembled with a resin-processed pen body (chisel shape) made of polyester fibers via a resin holder in such a manner that the front end of the axial barrel was in connection with the pen body, and a cap was fitted thereto to produce a marking pen. The rear end portion of the axial barrel has an SEBS resin attached as a friction member.

When an orange letter (handwriting) was formed by writing on a sheet of paper using the marking pen described above, the handwriting showed orange at room temperature (25°C), and the letter discolored and became colorless when the letter was rubbed using the friction member. This state could be maintained as long as the sheet of paper was not cooled to a temperature of -20°C or lower. Meanwhile, when the sheet of paper was put in a freezer and cooled to -20°C or lower, the letter showed a discoloration behavior of turning orange again, and the discoloration behavior was repeatedly reproducible.

### Application Example 7

### Preparation of reversibly thermochromic solid writing instrument

40 parts of the microcapsule pigment of Example 204, 35 parts of filler (talc), 10 parts of an excipient (side-chain crystalline polyolefin [manufactured by Hokoku Corporation, product name: HS Crysta 4100], 10 parts of an excipient (polyolefin wax) [manufactured by Sanyo Chemical Industries, Ltd., product name: SUNWAX 131-P (softening point: 110°C, Penetration: 3.5)], 2 parts of a styrene-acrylic acid copolymer, 2 parts of polyvinyl alcohol, and 1 part of a hindered amine-based light stabilizer were kneaded with a kneader to prepare a kneaded product for an inner core. Then, 69 parts of filler (talc), 10 parts of a sucrose fatty acid ester, 10 parts of an excipient (polyolefin wax), and 10 parts of an ethylene-vinyl acetate copolymer were kneaded with a kneader to prepare a kneaded product for an outer shell. The kneaded product for an outer shell was wound around an outer peripheral surface of the kneaded product for an inner core so that the kneaded product for an inner core became an inner core, and compression molding was performed by a press, the kneaded product was molded to have an outer diameter ϕ of 3 mm and a length of 60 mm (the inner core had a diameter ϕ of 2 mm, and cladding thickness of the outer shell was 0.5 mm), thereby producing a solid writing material having a core-sheath structure. The above dimensions are set values, and the solid writing material is manufactured by cooling to -20°C and returning to normal temperature after compression molding.

The solid writing material described above was stored and molded in a round outer shaft (wood shaft) to obtain a pencil. In addition, a cylindrical friction body made of an SEBS resin was secured to a rear end of the pencil with a metallic connecting member interposed therebetween to produce a solid writing instrument with a friction body (pencil with a friction body). When a green letter (handwriting) was formed by writing on a sheet of paper using the solid writing instrument described above, the handwriting showed green at room temperature (25°C), and the letter discolored and became colorless when the letter was rubbed using the friction member. This state could be maintained as long as the sheet of paper was not cooled to a temperature of -20°C or lower. Meanwhile, when the sheet of paper was put in a freezer and cooled to -20°C or lower, the letter showed a discoloration behavior of turning green again, and the discoloration behavior was repeatedly reproducible.

### Application Example 8

### Preparation of reversibly thermochromic writing instrument (reversibly thermochromic ballpoint pen)

A reversibly thermochromic liquid composition as an ink for writing instrument was prepared by mixing 25 parts of the microcapsule pigment of Example 206 (cooled to -20°C or lower in advance to develop blue), 0.3 parts of a shear thinning imparting agent (xanthan gum), 10 parts of urea, 10 parts of glycerin, 0.5 parts of a nonionic permeability imparting agent [manufactured by San Nopco Limited, product name: Nopco SW-WET-366], 0.1 parts of a modified silicone antifoaming agent [manufactured by San Nopco Limited, product name: Nopco 8034], 0.5 parts of a phosphoric acid ester surfactant [manufactured by DKS Co., Ltd., product name: PLYSURF AL], 0.5 parts of a pH regulator (triethanolamine), 0.2 parts of an antifungal agent [manufactured by Lonza Japan, product name: Proxel XL-2], and 52.9 parts of water.

The ink for writing instruments described above was sucked and filled in an ink-storing tube made of a polypropylene pipe and then connected, via a holder made of a resin, with a ballpoint pen tip holding a stainless steel ball having a diameter of 0.5 mm on its front end. Next, an ink follower (liquid plug) containing polybutene as a main component and having viscoelasticity was filled from the rear end of the ink-storing tube, and a tail plug was fitted in the rear portion of the pipe. A front axial barrel and a rear axial barrel were assembled, a cap was fitted thereto, and then degassing treatment was carried out by centrifugation, whereby a ballpoint pen was produced. The rear end portion of the rear axial barrel has an SEBS resin attached as a friction member.

When a blue letter (handwriting) was formed by writing on a sheet of paper using the ballpoint pen described above, the handwriting showed blue at room temperature (25°C), and the letter discolored and became colorless when the letter was rubbed using the friction member. This state could be maintained as long as the sheet of paper was not cooled to a temperature of -20°C or lower. Meanwhile, when the sheet of paper was put in a freezer and cooled to -20°C or lower, the letter showed a discoloration behavior of turning blue again, and the discoloration behavior was repeatedly reproducible.

### Application Example 9

### Production of reversibly thermochromic stamp

A reversibly thermochromic liquid composition as an ink for a stamp was prepared by mixing 20 parts of the microcapsule pigment of Example 207 (cooled to -20°C or lower in advance to develop blue), 50 parts of glycerin, 1.5 parts of an alkali-soluble acrylic emulsion [manufactured by Rohm and Haas Company Japan, product name: Primal DR 73], 0.9 parts of triethanolamine, 10 parts of a 50% aqueous polyvinylpyrrolidone solution, 0.2 parts of a silicone-based antifoaming agent, 0.5 parts of a permeation leveling agent, 0.2 parts of a preservative, and 16.7 parts of water.

The above-mentioned ink for a stamp was impregnated into a stamp material having continuous pores, and secured to a stamp main body so that the stamp face of the stamp material was exposed, and a cap was fitted to produce a stamp. An SEBS resin is attached as a friction member to a rear end portion of the stamp main body.

When the stamp described above was used and repeatedly pressed against a surface to be stamped (sheet of paper), the ink smoothly flowed out from the stamp face of the stamp material and transferred to the surface to be stamped, and a clear stamp image could be continuously formed without blurring the stamp image. The stamp image showed blue at room temperature (25°C), and discolored and became colorless when the stamp image was rubbed using the friction member. This state could be maintained as long as the image was not cooled to a temperature of -20°C or lower. Meanwhile, when the sheet of paper was put in a freezer and cooled to -20°C or lower, the stamp image showed the color change behavior of turning blue again, and the color change behavior was repeatedly reproducible.

### Application Example 10

### Preparation of reversibly thermochromic printed matter (reversibly thermochromic T-shirt)

30 parts of the microcapsule pigment of Example 208 (cooled to -20°C or lower in advance to develop red) was uniformly mixed in an aqueous vehicle composed of 60 parts of acrylic emulsion (solid content: 45%), 0.2 parts of an antifoaming agent, 1 part of a viscosity adjusting agent, and 8.8 parts of water to prepare a reversibly thermochromic liquid composition as a printing ink.

A large number of heart patterns were printed with the printing ink described above using a 100 mesh screen plate on a white T-shirt (made of cotton) as a support, and the printing ink was dried and cured to provide a reversibly thermochromic layer, thereby preparing a reversibly thermochromic printed matter (reversibly thermochromic T-shirt).

In the T-shirt, a large number of red heart patterns were visually recognized on the surface of the T-shirt at room temperature (25°C), and the heart patterns were not changed by the body temperature or ambient temperature; however, when the T-shirt was heated to 58°C or higher, a portion where the heart patterns were printed turned colorless, and the red heart patterns were not visually recognized. When the T-shirt was cooled to -20°C or lower, a red heart pattern was visually recognized again. This change could be repeated.

It was possible to arbitrarily change the design of the T-shirt by decoloring some of the heart patterns on the surface of the T-shirt by heating with an iron or the like, to form a pattern in which an arbitrary heart pattern alone was decolored. The discolored state could be maintained at room temperature (25°C), and by heating the whole T-shirt to 58°C or higher to decolor all of the heart patterns and then cooling the T-shirt to -20°C or lower, it was possible to allow the heart patterns to develop the color again.

### Application Example 11

### Production of reversibly thermochromic plug

2.5 parts of the microcapsule pigment of Example 209 (cooled to -20°C or lower in advance to develop blue-green) and 1.5 parts of a general orange pigment were stirred and mixed in an oily vehicle composed of 12.5 parts of a vinyl chloride-vinyl acetate copolymer, 38.3 parts of xylene, 45 parts of butyl acetate, and 0.2 parts of a viscosity modifier to prepare a reversibly thermochromic liquid composition which was a coating material used for spray coating.

A reversibly thermochromic layer was provided on a plug portion (white) of a household electric cord as a support by spray coating using the above coating material and drying to produce a reversibly thermochromic plug.

The reversibly thermochromic plug showed brown at room temperature (25°C), and when the plug turned orange at a temperature of 59°C or higher, it was capable of maintaining a discolored state in orange unless it was cooled to -20°C or lower; therefore, a temperature history when the plug was overheated and reached a high-temperature range of 59°C or higher could be visually confirmed.

### REFERENCE SIGNS LIST

- t₁: complete coloring temperature
- t₂: coloring starting temperature
- t₃: decoloring starting temperature
- t₄: complete decoloring temperature
- T₁: complete decoloring temperature
- T₂: decoloring starting temperature
- T₃: coloring starting temperature
- T₄: complete coloring temperature
- ΔH: hysteresis width

## Claims

1. A reversibly thermochromic composition comprising:
(a) an electron-donating color-developing organic compound;
(b) a combination of a compound represented by Formula (I) and a compound represented by Formula (IIa) or (IIb) as an electron-accepting compound; and
(c) a reaction medium which reversibly induces an electron transfer reaction between the component (a) and the component (b) in a specific temperature range: 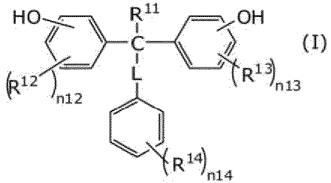
wherein
R¹¹ is a hydrogen atom or a linear or branched alkyl group having 1 to 6 carbon atoms,
L is a single bond, a linear or branched alkylene group having 1 to 3 carbon atoms, or an aryl-substituted alkylene group having 7 to 9 carbon atoms,
R¹² and R¹³ are each independently a linear or branched alkyl group having 1 to 4 carbon atoms, which may be substituted by a fluorine atom, a cyclic alkyl group having 3 to 7 carbon atoms, a linear or branched alkoxy group having 1 to 3 carbon atoms, an alkenyl group having 2 to 4 carbon atoms, an aryl group having 6 to 10 carbon atoms, or a halogen atom,
R¹⁴ is each independently a linear or branched alkyl group having 1 to 4 carbon atoms, which may be substituted by a fluorine atom, a cyclic alkyl group having 3 to 7 carbon atoms, a linear or branched alkoxy group having 1 to 3 carbon atoms, an alkenyl group having 2 to 4 carbon atoms, an aryl group having 6 to 10 carbon atoms, which may be substituted by a halogen atom or a hydroxy group, an aryl-substituted alkyl group having 7 to 11 carbon atoms, which may be substituted by a halogen atom or a hydroxy group, a hydroxy group, or a halogen atom,
n12 and n13 are each independently 0 to 3, and
n14 is 0 to 3, 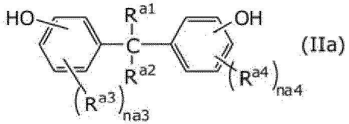 wherein
R^{a1} and R^{a2} are each independently a hydrogen atom or a linear or branched alkyl group having 1 to 17 carbon atoms (where a methylene (-CH₂-) group in the alkyl group may be replaced with an oxy (-O-) group or a carbonyl (-CO-) group), which may be substituted by a fluorine atom,
R^{a3} and R^{a4} are each independently a linear or branched alkyl group having 1 to 4 carbon atoms, which may be substituted by a fluorine atom or a hydroxy group, an alkenyl group having 2 to 4 carbon atoms, or a halogen atom, and
na3 and na4 are each independently 0 to 2, 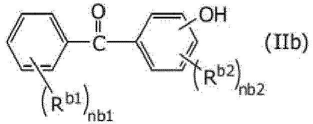 wherein
R^{b1} and R^{b2} are each independently a hydroxy group, a linear or branched alkoxy group having 1 to 9 carbon atoms, a linear or branched alkyl group having 1 to 10 carbon atoms, which may be substituted by a fluorine atom, an alkenyl group having 2 to 10 carbon atoms, or a halogen atom,
nb1 is 0 to 3, and
nb2 is 0 to 2.

2. The composition according to claim 1, comprising, as the component (b), a combination of the compound represented by Formula (I) and the compound represented by Formula (IIa).

3. The composition according to claim 1 or 2, wherein in Formula (I), when R¹¹ is a hydrogen atom or a methyl group, L is a single bond or an ethylene group, n12 and n13 are each 0, n14 is 0 or 1, and n14 is 1, R¹⁴ is a methyl group, an ethyl group, a cyclohexyl group, a methoxy group, an ethoxy group, a hydroxyphenyl-substituted propyl group, or a hydroxy group, and each hydroxy group is present at the 4-position of a benzene ring.

4. The composition according to claim 2 or 3, wherein in Formula (IIa), at least one of R^{a1} and R^{a2} is a linear or branched alkyl group having 5 to 9 carbon atoms.

5. The composition according to any one of claims 2 to 4, wherein a mass ratio of the compound represented by Formula (I) : the compound represented by Formula (IIa) is 1 : 0.5 to 1 : 5.

6. A reversibly thermochromic microcapsule pigment encapsulating the composition according to any one of claims 1 to 5.

7. A reversibly thermochromic liquid composition comprising the reversibly thermochromic microcapsule pigment according to claim 6 and a vehicle.

8. The reversibly thermochromic liquid composition according to claim 7, which is selected from the group consisting of a printing ink, an ink for writing instruments, an ink for coating tools, an ink for a stamp, an ink for ink jet use, a paint, an ultraviolet curable ink, a painting color, a cosmetic material, and a coloring liquid for fibers.

9. A solid writing material or a solid cosmetic material comprising: the reversibly thermochromic microcapsule pigment according to claim 6; and an excipient.

10. A resin composition for forming a reversibly thermochromic molded article comprising the reversibly thermochromic microcapsule pigment according to claim 6 and a molding resin.

11. A reversibly thermochromic molded article obtained by molding the resin composition for forming a reversibly thermochromic molded article according to claim 10.

12. A reversibly thermochromic laminate comprising: a support; and a reversibly thermochromic layer comprising the reversibly thermochromic microcapsule pigment according to claim 6.

13. A writing instrument comprising storing the reversibly thermochromic liquid composition according to claim 7.

14. The writing instrument according to claim 13, comprising a friction member that changes color of a handwriting of the writing instrument by frictional heat.
